# EUROPEAN PATENT SPECIFICATION

(11) **EP 0 557 345 B1**
(45) Date of publication and mention of the grant of the patent: **14.09.1994**
(21) Application number: 91919864.8
(22) Date of filing: 15.11.1991
(51) Int. Cl.: C07C 237/46, A61K 49/04

(54) **1,3-BIS- 3-(MONO- OR POLY-HYDROXY)ACYLAMINO-5-(MONO- OR POLY-HYDROXYALKYL)AMINOCARBONYL-2,4,6-TRIIODO-BENZOYL-AMINO]-HYDROXY- OR HYDROXYALKYL-PROPANES, THEIR METHODS OF PREPARATION AND X-RAY CONTRAST MEDIA CONTAINING THEM**
1,3-BIS-[3-(MONO-[ODER POLYHYDROXY)ACYLAMINO-5-(MONO- ODER POLYHYDROXYALKYL)AMINOCARBONYL-2,4,6-TRIJODOBENZOYLAMINO]-HYDROXY- ODER HYDROXYALKYL-PROPANE, DEREN HERSTELLUNG UND DIESE ENTHALTENDE RÖNTGENKONTRASTMITTEL
1,3-BIS- 3-(MONO-[OU POLY-HYDROXY)ACYLAMINO-5-(MONO- OU POLY-HYDROXYALKYLE)AMINOCARBONYLE-2,4,6-TRIIODO-BENZOYLE-AMINO]-HYDROXY- OU HYDROXYALKYLE-PROPANES, PROCEDES DE PREPARATION ET MILIEUX DE CONTRASTE DE RAYONS X LES CONTENANT

(30) Priority: 16.11.1990 IT 2208890
(43) Date of publication of application: 01.09.1993
(73) Proprietor: DIBRA S.p.A., I-20122 Milano (IT); BRACCO S.p.A., 20134 Milano (IT)
(72) Inventor: UGGERI, Fulvio, I-20134 Milano (IT); BROCCHETTA, Marino, I-20134 Milano (IT)
(74) Representative: Minoja, Fabrizio
(86) International application number: EP9102151
(87) International publication number: WO9208691

(56) References cited:
- EP-A- 0 308 364
- WO-A-85/01727
- DE-A- 2 805 928
- US-A- 4 062 934

## Description

This invention relates to symmetrical or asymmetrical 1,3-bis-[3-(mono- or poly-hydroxy)acylamino-5-(mono- or polyhydroxyalkyl)aminocarbonyl-2,4,6-triiodo-benzoyl-amino]-hydroxy- or hydroxyalkyl-propanes, useful to constitute the opacifying component of X-ray contrast media, of general formula (I) 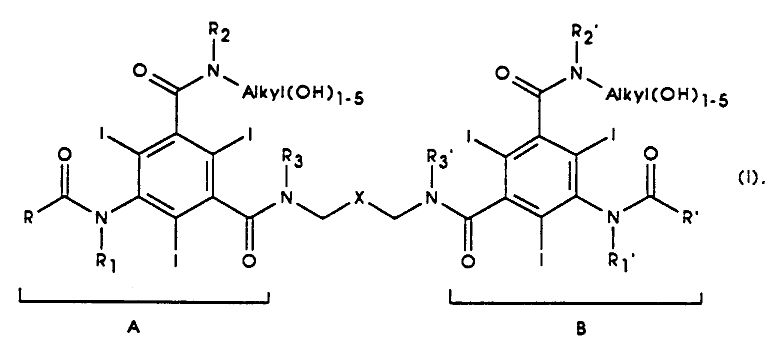
wherein:
- R, R',: which are the same or different, are a straight or branched mono- or poly-hydroxyalkyl C₁-C₃ residue containing from 1 to 2 OH groups,
- R₁, R₂, R₃,: which are the same or different, are H or CH₃,
- R_{1'}, R_{2'}, R_{3'},: which are the same or different, are H or CH_{3,}
- Alkyl(OH)₁₋₅: is one of the groups of formula -CH(CH₂OH)CH(OH)CH₂OH, -CH(CH₂OH)₂, -CH₂CH(OH)-CH₂OH, -CH₂(CHOH)₄CH₂OH, or -CH₂CH₂OH,
- X: is one of the groups -CH(OH)-, -CH(CH₂OH)-, -C(OH)(CH₂OH)- or -C(CH₂OH)₂-,
- A and B,: may be the same or different.

The invention also comprises the possible enantiomers, diastereoisomers and/or rotamers of compounds (I).

The invention also relates to a process for the preparation of these non-ionic compounds, as well as to the X-ray contrast media which contain them as opacifying components.

Preferred compounds of formula (I) are the ones in which R₂, R₂', R₃, R₃', are H. Particularly preferred are the ones in which X represents the -CH(OH)- group. From the structural point of view, the non-ionic X-ray contrast agents which are more similar to the ones of this invention are the a,ω-bis-[3-hydroxyacylamino-5-(dihydroxyalkyl)aminocarbonyl-2,4,6-triiodo-benzoyl-amino]-oxaalkanes described in patents DE 2805928 and US 4,139,605. In these compounds the two triiodo-benzoyl-amino residues are joined via an oxaalkylenic residue of formula -CₙH₂ₙ-(O-CₙH₂ₙ)₀₋₄-O-CₙH₂ₙ- containing from 4 to 12 atoms of C and from 1 to 5 atoms of O. They can produce oversaturated solutions and after some time, these ones may spontaneously crystallize, therefore causing a limitation to the use of such compounds. Patent US 4,062,934 describes N,N'-bis-(3-N-methyl-N-acetylamino-5-N-gluconylamino-2,4,6-triiodo-benzoyl)-diaminoalkanes. Due to an iodine content comparatively low, they show a reduced opacifying capacity. In addition, their solutions are quite viscous. These features cause some problems when the product is used in procedures which require administration via catheter.

N,N'-bis-(3-acylamino-5-N-methyl-carbamoyl-2,4,6-triiodo-benzoyl-amino)-carboxyalkanes are described in patent application GB 1,488,904. The preparation of solutions acceptable from the pharmacological point of view requires that free carboxylic groups, where present, are neutralized. Therefore, if compared to the non-ionic contrast agents, the solutions of the compounds described in the above mentioned patent application have a high osmolality. Similarly, their neurotoxicity is higher than the one of the non-ionic contrast media.

Sovak et Al. described in application WO 8501727, among other compounds, also N,N'-bis-[3,5-bis-(N-2,3-dihydroxy-propyl-N-acetylamino)-2,4,6-triiodo-benzoyl]-ethylendiamines and the corresponding N'-hydroxyalkyl-derivatives. The synthesis of these compounds is quite difficult. The four 2,3-dihydroxy-propyl groups are obtained by treating the corresponding tetraalkenylderivatives with 1-butyl-hydroperoxide in the presence of osmium tetroxide. The reaction occurs in a fragmented and unaccomplished way. None of the compounds described, mentioned or claimed by Sovak has as conjunction bridge of the two 2,4,6-triiodo-benzoyl-amino residues the hydroxyalkylenic chain of formula -CH₂-X-CH₂-, with X equivalent to -CH(OH)-, -CH(CH₂OH)-, -C(OH)(CH₂OH)-or -C(CH₂OH)₂-, which is characteristic of this invention.

A last reference concerns the European patent applications EP 74307 and EP 74309 abandoned in 1985.

The first one only describes aromatic i- or tricyclic compounds where the aromatic moiety contains iodine and bromine atoms at the same time and in the same molecule.

The second one does not describe new compounds, but a process which increases the tolerability of opacifying compositions by using a mixture of iodobenzenic and bromobenzenic compounds. The general formulas describing these compounds are extremely wide, unclear and indefinite and include billions of compounds basically different among them.

The compounds of formula (I) belong to the class of non-ionic contrast agents for X-ray diagnosis, which are more and more replacing the salts up to now used of the derivatives of 2,4,6-triiodo-benzoic acid, due to their better tolerability and the reduced side-effects. Thanks to the elimination of the ionic species, their solutions, if compared to the ones of the ionic agents, have the same content of iodine, but a lower osmotic pressure, that's to say a lower osmolality. Therefore, for instance, in angiography, they cause less pain and endothelial damage. In subarachnoidal administration for myelography and cisternography, unlike the previously used contrast media, they rarely cause arachnoiditis or epileptic disorders. Unfortunately non-ionic contrast agents, consisting of iodinated monocyclic aromatic nuclei, are still too hypertonic, despite their excellent physical and pharmacological properties, if compared with blood, at the high dosages and high concentrations which many diagnoses require. This fact led to the development of bicyclic hexaiodinated compounds, whose osmolality is reduced in comparison with the total amount of iodine in the molecule. However, concentrated solutions of these compounds are frequently too much viscous. In addition, a large number of products, even if they are expected to be promising from the theoretical point of view, are not enough soluble.

For this reason, researchers investigated on new hexaiodinated bicyclic derivatives characterized by: high solubility, low viscosity and osmolality as well as high intravenous, intracisternal and intracerebral tolerability and minimum tendency to give side effects (pain, temperature increase, nausea, decrease of pressure and vessel damages).

In particular, physicians need new contrast media characterized by a wide range of possible uses, i.e. in urography, angiography, cardioangiography, flebography, myelography, cisternography, lymphography, isterosalpingography, bronchography and gastrography or the visualization of articular cavities.

The products of this invention are generally characterized by a good water solubility, which can exceed 100 g per 100 ml of solution at 20°C, and a low toxicity.

If compared to the known hexaiodinated dimers, they show low osmolality without causing a foreseeable corresponding rise in the viscosity.

One of the preferred compounds of the invention is for example 1,3-bis-[3-(L-2-hydroxy-propionyl)amino-5-(1,3-dihydroxy-isopropyl)aminocarbonyl-2,4,6-triiodo-benzoyl-amino]-2-hydroxy-propane (compound A of Table 2). It gives stable aqueous solutions which contain, at 20°C, more than 100 g of product in 100 ml of solution.

As far as the physiological characteristics are concerned, the study of plasma kinetics revealed half life for the distribution and elimination phases of 2.9 and 22.9 min, after intravenous administration in rats (200 mgI/kg).

The apparent volume of distribution is 123.5 ml/kg showing that the product is distributed in plasma and the extracellular spaces. The total clearance is 7.9 ml . min^{-1.}. kg⁻¹.

Elimination occurs mainly through urinary pathway and in less extent through the bile. After 7 hours from the administration, 76.7% of the administered dose was found in the urine and 3.5% in the bile.

Its tolerability is very good as reported in Table 1.

In Table 2, there are reported the values of some characteristic properties of preferred products of this invention in comparison with IODIXANOL (Nycomed), which is an hexaiodinated non-ionic dimer in advanced phase of development (EP 108638; CLINICA 413, p 7, 08.08.90).

The process for the preparation of the 1,3-bis-[3-(mono- or poly-hydroxy)acylamino-5-(mono- or poly-hydroxyalkyl)aminocarbonyl-2,4,6-triiodo-benzoyl-amino]-hydroxy- or hydroxyalkyl-propanes of general formula (I) is characterized in that a 1,3-diamino-hydroxy- or hydroxyalkyl-propane of general formula (II)

R₃-HN-CH₂-X-CH₂-NH-R₃' (II)

wherein R₃ and R₃' are H or CH₃, X is one of the groups -CH(OH)-, -CH(CH₂OH)-, -C(OH)(CH₂OH)- or -C(CH₂OH)₂- and one of the amino groups may be protected by a suitable protective group, is reacted, directly or by a multi-step process, with a reactive derivative of a 3-(mono- or poly-acyloxy)aclyamino-5-(mono- or poly-hydroxyalkyl)aminocarbonyl-2,4,6-triiodo-benzoic acid of general formula (III) 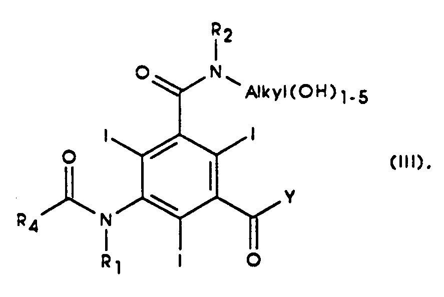
wherein:
- R₁, R₂,: are H or CH₃,
- R₄,: is a straight or branched mono- or poly-acyloxyalkyl C₁-C₁₃ residue containing from 3 to 13 atoms of C and from 1 to 2 lower acyloxy groups C₂-C₅,
- Alkyl(OH)₁₋₅,: is one of the groups of formula -CH₂CH(OH)CH₂OH, -CH₂CH₂OH, -CH(CH₂OH)₂, -CH(CH₂OH)CH(OH)CH₂OH or -CH₂(CHOH)₄CH₂OH, where the hydroxy groups may be preferably protected by acetalic or ketalic groups,
- CO-Y,: is the residue of a mixed anhydride or, preferably, a halocarbonyl group,
to give a compound of general formula (IV) 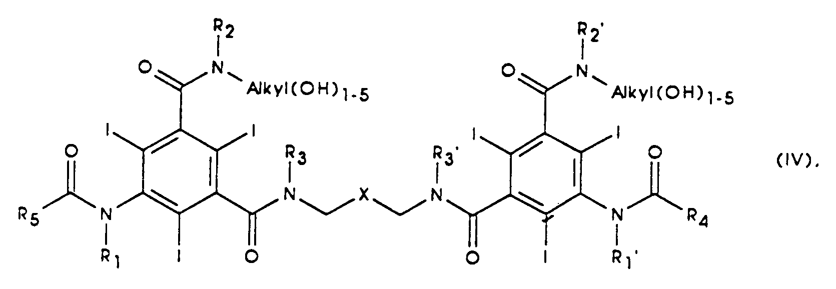
wherein R₁, R₂, R₃, R₁', R₂', R₃', R₄, Alkyl(OH)₁₋₅, and X have the meaning as previously defined and R₅ may be R or R₄, preferably according to one of the two following procedures:
a) a compound of formula (II) is reacted with a compound of formula (III) in a molar ratio of 1:2 in a solvent and in the presence of a basic condensation agent in order to obtain the corresponding compound of formula (IV) where R₅ corresponds to R₄,
b) a compound of formula (II), where one of the two amino groups is protected by a suitable protective group, is reacted with a compound of formula (III) in a molar ratio of 1:1 in a solvent and in the presence of a basic condensation agent in order to obtain, after hydrolysis of the protective groups, the corresponding 1-[3-(mono- or poly-hydroxy)-acylamino-5-(mono- or poly-hydroxyalkyl)aminocarbonyl-2 4,6-triiodo-benzoyl-amino]-3-aminohydroxyalkyl derivative of general formula (V)

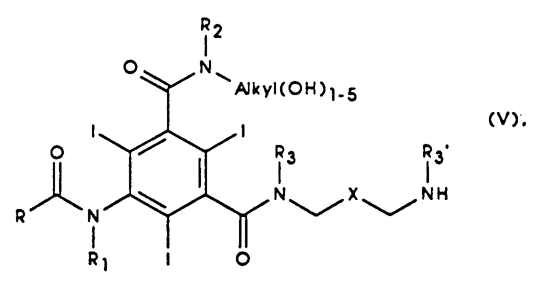

wherein R, R₁, R₂, R₃, R₃', Alkyl(OH)₁₋₅ and X have the meaning as previously defined, and this compound (V) is subsequently reacted with a derivative of formula (III) in the presence of a basic condensation agent to obtain compound (IV) where R₅ is R, then, the compound of formula (IV), obtained by synthetic methods a) or b), is transformed by hydrolysis of the protective groups, into the corresponding compound of formula (I). Finally, in case that R₁, R₁' are H, this last one may, if desidered, be methylated in alkaline medium.

Methylation occurs through reaction with suitable methylating agents, for instance methyl halides, dimethylsulphate, methylsulphonate, dimethylcarbonate. For instance, the di-sodium derivative of general formula (VI) 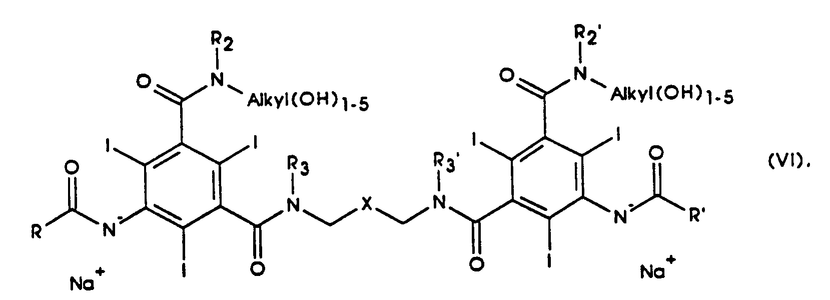
is prepared in a basic environment, preferably in the presence of alcoholate or alkaline hydroxide, and is then transformed into the corresponding dimethyl derivative of general formula (VII) 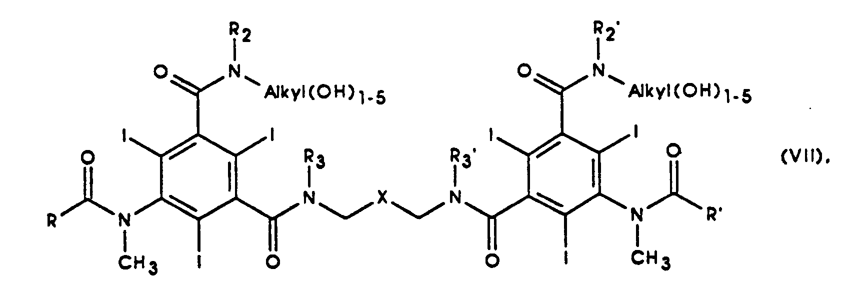
by treatment with methyl halides, dimethylsulphate, dimethyl carbonate, methyl methanesulphonate, methyl benzenesulphonate, methyl toluenesulphonate.

In case of direct reaction of the product of general formula (II) with a compound of general formula (III), according to synthesis a), symmetrical products are obtained, where both residues A and B of general formula (I) are identical.

Following the multistep reaction according to synthesis b), asymmetrical products may be obtained, in which the residues A and B of general formula (I) are different. In fact, during the second step of the reaction, a compound of formula (III), which is different from the one used during the first step of the reaction, may be used.

Anhydrous tertiary amines, potassium hydroxide, sodium hydroxide, sodium bicarbonate, calcium carbonate, magnesium carbonate may be used as basic condensation agents.

In both a) and b) synthesis, anhydrides with organic or inorganic acids, azides, derivatives of phosphoric acids, alkylcarbonic acids may be used as compounds of formula (III).

Therefore, the reactive residue Y in compounds of formula (III) represents the residue of an inorganic or organic acid such as: chlorine, bromine, iodine, phosphite, azide, acyloxy or alkoxycarbonyloxy.

The preferred group CO-Y is the residue CO-Cl.

The hydroxy functions in the Alkyl(OH)₁₋₅ group of formula (III) may be preferably protected by transformation into the corresponding acetales or ketals. Examples of such protective groups may be for instance methylidene, ethylidene, 1-methyl-ethylidene, 1-ethyl-ethylidene, 1-t-butyl-ethylidene, 1-phenyl-ethylidene, 2,2,2-trichloro-ethylidene, 1-ethyl-propylidene. These groups may be easily and fully submitted to hydrolysis through a short treatment with a strong acid, for instance diluted hydrochloric acid, aqueous trifluoroacetic acid or through a strongly acidic ion exchange resin, releasing the corresponding ketonic compounds, usually acetone, methylethylketone or diethylketone.

The lower acyloxy protective C₂-C₅ groups, included in the R₄ residue of formula (III), may be for instance acetoxy, propionyloxy, butyroyloxy and may be easily submitted to hydrolysis by treatment with alkaline aqueous solutions, in order to obtain the desired mono- or poly-hydroxyalkyl R groups of formula (I).

The reaction of a compound of general formula (II) with one of formula (III) to give a compound (IV) takes place preferably in an aprotic solvent, such as, for instance, dimethylacetamide (DMAC), dimethylformamide (DMF), hexamethylphosphoramide (HMPT) or dioxane, but also in acetone, ethyl alcohol and isopropyl alcohol. The temperature is not critical. The reaction takes place when temperature ranges from -10°C to + 150°C, preferably within 0°C and 100°C.

1,3-Diamino-hydroxy- or hydroxyalkyl-propanes of general formula (II) are described in literature, for instance in "Beilsteins Handbuch der Organischen Chemie":
1,3-diamino-2-hydroxy-propane, Beil.4 H 290, E II 739, E III 766, E IV 1694;
1,3-bis-(methylamino)-2-hydroxy-propane, Beil. 4 E IV 1695;
1,3-diamino-2,2-bis-hydroxymethyl-propane, Beil. 4 E III 850;
1,3-bis-(methylamino)-2,2-bis-hydroxymethyl-propane, Beil. 4 E III 851.

When a compound of formula (II) is reacted with one of formula (III) in a molar ratio of 1:1 according to synthesis b), then the 1,3-diaminoderivative of formula (II) is monoprotected on one of the two amino groups in order to avoid side-reactions. Protective groups which meet this purpose may be for instance acetyl, dichloroacetyl, trifluoroacetyl groups. Trifluoroacetyl is particularly preferred. Afterwards, these groups may be easily and completely hydrolysed through treatment with alkaline aqueous solutions.

The reactive derivatives of the 3-(mono- or poly-acyloxy)acylamino-5-(mono- or poly-hydroxyalkyl)amino-2,4,6-triiodo-benzoic acids of general formula (III) are obtained by reaction of a reactive derivative of a 3-(mono- or poly-acyloxy)acylamino-2,4,6-triiodo-isophthalic acid of general formula (VIII) 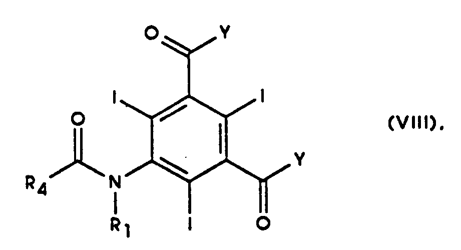
wherein R₁, R₄, and Y are as previously defined, or by reaction of a derivative belonging to the ones already described in patents US 4,001,323 and EP 26281 with the hydroxyalkyamines 2-hydroxy-ethylamine, 1,3-dihydroxy-isopropylamine (serinol), 2,3-dihydroxy-propylamine (isoserinol), 3-amino-1,2,4-butantriol, or with the corresponding N-methylamines, or with N-methyl-glucamine or with a corresponding derivative thereof wherein the hydroxy groups are preferably protected by chelatization, for instance with 5-amino-2,2-dimethyl-1,3-dioxane, 5-amino-2-methyl-2-ethyl-1,3-dioxane, 5-amino-1,3-dioxane, 5-amino-2,2-diethyl-1,3-dioxane, 4-aminomethyl-2,2-dimethyl-1,3-dioxolane, 4-aminomethyl-2,2-diethyl-1,3-dioxolane, 5-amino-6-hydroxy-2,2-dimethyl-1,3-dioxepane or 5-amino-6-hydroxy-2-ethyl-2-methyl-1,3-dioxepane.

The reactive derivatives of 3-(mono- or poly-acyloxy)acylamino-2,4,6-triiodo-isophthalic acid (VIII) are generally reacted with one of the above mentioned amines in a molar ratio of about 1:2 without using a basic condensation agent, or in a ratio of 1:1 by using a basic condensation agent in an inert organic solvent. For instance, dioxane or tetrahydrofuran (THF) are preferred solvents. The HY acid, released during the reaction of (VIII) with the amine, is neutralized by the second mole of the amine, giving the corresponding ammonium salt, or by the basic condensation agent used.

Some of the preferred reactive derivatives of the 3-(mono- or poly-acyloxy)acylamino-5-(mono- or poly-hydroxyalkyl)aminocarbonyl-2,4,6-triiodo-benzoic acids of general formula (III) have already been described in patents DE 2805928 or US 4,139,605.

The compounds of this invention may be used as opacifying agents in non-ionic contrast media for X-ray diagnosis.

They may be formulated in a suitable carrier agent which is acceptable from the pharmacological point of view. Suitable carrier agents include, for instance, the ones which can be used for enteral or parenteral administration, such as buffered sterile aqueous solutions containing tromethamine, phosphate, citrate and/or bicarbonate ions, ionically balanced solutions containing physiologically acceptable anions and cations such as: Cl⁽⁻⁾, HCO₃⁽⁻⁾, Ca⁽²⁺⁾, Na⁽⁺⁾, K⁽⁺⁾ and Mg⁽²⁺⁾. Solutions of the contrast agents of this invention may also contain a small amount of a physiologically tolerable chelating agent, such as the sodium and calcium salts of EDTA, in concentrations from 0,05 to 2 mM/l, or even heparin, at a dosage ranging from 5 to 500 units per 100 ml of solution, or another suitable anticoagulant agent.

Hypotonic solutions of the contrast media of this invention may be isotonically balanced by adding the correct amount of Merlis liquid (The Am. J. of Phys. Vol. 131, 1940 p. 67-72). Useful concentrations of iodine for such contrast media vary from 140 to 500 mgl/ml at a dosage of 10-300 ml, according to the desired diagnostic use.

The following experimental examples show the basic principles of this invention.

### EXAMPLE 1

### 1,3-bis-[3-(L-2-hydroxy-propionyl)amino-5-(1,3-dihydroxy-isopropyl)-aminocarbonyl-2,4,6-triiodo-benzoyl-amino]-2-hydroxy-propane.

a) 124 g of 3-(L-2-acetoxy-propionyl)amino-5-(1,3-dihydroxy-isopropyl)aminocarbonyl-2,4,6-triiodo-benzoyl-chloride (0.162 mol), obtained according to the procedure described in DE 2805928/Example 1, are solubilized in 490 ml of dimethylformamide (DMF). The resulting solution is cooled to 0-5°C, added of 32.4 g of anhydrous tributylamine (0.175 mol) and then dropwise mixed under stirring with a solution of 7.66 g of 1,3-diamino-2-hydroxy-propane (0.085 mol) in 200 ml of DMF. The reaction mixture is kept under stirring at 0-5°C for 1 h, then is left at room temperature for about 20 h. Hence the solvent is evaporated under vacuum. The residue is crystallized with ethyl acetate. The crystalline precipitate is filtered and dried, then suspendend in 600 ml of water at 50°C. pH is adjusted at 10.3 with NaOH 2N and the mixture is stirred until the acetoxy groups of the propionyl residues are totally hydrolysed. The resulting solution is made free from salts by passage through ion exchange resins (360 ml of Amberlite^{R} IR 120 and 400 ml of Duolite^{R} A 30B). The eluate is evaporated to dryness and crystallized from 600 ml of ethyl alcohol. After filtration and drying 75.8 g of 1,3-bis-[3-(L-2-hydroxy-propionyl)amino-5-(1,3-dihydroxy-isopropyl)aminocarbonyl-2,4,6-triiodo-benzoyl-amino]-2-hydroxy-propane are obtained.

| | | | |
|---|---|---|---|
| Yield: | 64% | m.p.: | 280°C (dec.) |

| Elemental Analysis (%) | | | | |
|---|---|---|---|---|
| | C | H | I | N |
| Calculated: | 25.47 | 2.48 | 52.08 | 5.76 |
| Found: | 25.47 | 2.35 | 52.12 | 5.53 |

[α]²⁰₄₃₆ = -6.03° (c = 9.6% H₂O)
Solubility in H₂O at 20°C : > 100% w/v.
- TLC:: (Silica Gel 60 F₂₅₄, Merck)
- Eluent:: CHCl₃/CH₃OH/NH₄OH 25% = 6/3/1 v/v/v. Rf = 0.14.
- HPLC:: Rt = 12.4 min. Titre = 98.5% (on the area)

### Chromatographic conditions:

- Column:: LICHROSORB^{R}RP 18 (Merck), 5 µ (250 x 4 mm)
- Eluent:: A) H₂O B) CH₃CN

Gradient (flow 1 ml/min; Detector UV 254 nm):

| | | | | |
|---|---|---|---|---|
| time (min) | = | 0 - 3; | 3 - 25; | 25 - 30 |
| %B | = | 0; | 0 - 40; | 40 |

b) the above disclosed compound can be obtained also by reacting, according to the procedure of the previous Example 1a), 65.2 g of 3-(L-2-acetoxy-propionyl)amino-5-(4,4-dimethyl-3,5-dioxa-cyclo-hexyl)aminocarbonyl-2,4,6-triiodo-benzoylchloride (0.081 mol), described in DE 2805928/Example 1B(b), with 3.83 g of 1,3-diamino-2-hydroxy-propane (0.042 mol) in 350 ml of DMF in the presence of 16.2 g of tributylamine (0.087 mol). The obtained product is transformed into 1,3-bis-[3-(L-2-acetoxy-propionyl)amino-5-(1,3-dihydroxy-isopropyl)aminocarbonyl-2,4,6-triiodo-benzoyl-amino]-2-hydroxy-propane by treatment with HCl 2N and corresponding elimination of acetone. This product is suspended in water at 50°C, the pH is adjusted to 10.3 with NaOH 2N and the basic treatment continues until the acetoxy groups are completely hydrolysed. After elimination of the ionic species, due to the passage through ion exchange resins, 41.4 g of 1,3-bis-[3-(L-2-hydroxy-propionyl)amino-5-(1,3-dihydroxy-isopropyl)aminocarbonyl-2,4,6-triiodo-benzoyl-amino]-2-hydroxy-propane are obtained with a yield of 70%.
c) This reaction may also be performed in ethyl alcohol (EtOH) instead of DMF: 588 g of 3-(L-2-acetoxy-propionyl)-amino-5-(1,3-dihydroxy-isopropyl)aminocarbonyl-2,4,6-triiodo-benzoyl-chloride (0.67 mol) in 5.8 l of EtOH are reacted at room temperature with 36.4 g of 1,3-diamino-2-hydroxy-propane (0.404 mol) dissolved in 2.9 l of EtOH, in the presence of 144 g of tributylamine (0.777 mol). The obtained precipitate is filtered, suspended in water and treated with NaOH 2N up to pH 10.3 as previously described in a). 350 g of 1,3-bis-[3-(L-2-hydroxy-propionyl)-amino-5-(1,3-dihydroxy-isopropyl)aminocarbonyl-2,4,6-triiodo-benzoyl-amino]-2-hydroxy-propane are obtained with a yield of 62.2%.

### EXAMPLE 2

### 1,3-bis-[3-hydroxyacetylamino-5-(1,3-dihydroxy-isopropyl)aminocarbonyl-2,4,6-triiodo-benzoyl-amino]-2,2-bis-hydroxymethyl-propane.

a) 144 g of 3-acetoxyacetylamino-5-(1,3-dihydroxy-isopropyl)aminocarbonyl-2,4,6-triiodo-benzoyl-chloride (0.192 mol), described in DE 2805928/Example 10, dissolved in 600 ml of DMF, are dropwise added under stirring and between 5 to 10°C to a solution of 13.4 g of 1,3-diamino-2,2-bis-hydroxymethyl-propane (0.10 mol) and 39 g of tributylamine (0.21 mol) in 80 ml of DMF. The reaction mixture is kept under stirring for some hours at 10-20°C. Hence it is poured under heavy stirring into 5.5 l of ethyl acetate from which the final product crystallizes. After filtration and drying, 140 g of raw 1,3-bis-[3-acetoxyacetylamino-5-(1,3-dihydroxy-isopropyl)aminocarbonyl-2,4,6-triiodo-benzoyl-amino]-2,2-bis-hydroxymethyl-propane are obtained.

A sample crystallized from methyl alcohol has the following characteristics:
- m.p.:: 284-287°C

| Elemental Analysis (%) | | | | |
|---|---|---|---|---|
| | C | H | I | N |
| Calculated: | 26.91 | 2.58 | 48.74 | 5.37 |
| Found: | 26.82 | 2.70 | 48.45 | 5.37 |

b) 105 g of raw 1,3-bis-[3-acetoxyacetylamino-5-(1,3-dihydroxy-isopropyl)aminocarbonyl-2,4,6-triiodo-benzoyl-amino]-2,2-bis-hydroxymethyl-propane are diluted in 1.9 l of water and 0.8 l of methyl alcohol (CH₃OH) and slowly mixed with 170 ml of NaOH 1N until a constant pH value of 10.3 is obtained. The mixture is kept under stirring for about 10 h at 20°C. Then, the methyl alcohol is evaporated under vacuum. The resulting aqueous solution is made free from salts by passage through ion exchange resins (160 ml of Amberlite^{R} IR 120 and 150 ml of Duolite^{R} A 30B). The eluate is evaporated to dryness under vacuum, diluted again in 1.2 l of water, bleached on active carbon and percolated on 1200 ml of Amberlite^{R} XAD-2. The fractions containing the product are gathered and dried. 66 g of 1,3-bis-[3-hydroxyacetylamino-5-(1,3-dihydroxy-isopropyl)aminocarbonyl-2,4,6-triiodo-benzoyl-amino]-2,2-bis-hydroxymethyl-propane are obtained.

The last impurities are eliminated by dissolving the product in aqueous ethyl alcohol 80% and filtering the turbid solution.

| | | | |
|---|---|---|---|
| Yield: | 62% | m.p.: | 285°C |

| Elemental Analysis (%) | | | | |
|---|---|---|---|---|
| | C | H | I | N |
| Calculated: | 25.19 | 2.45 | 51.51 | 5.68 |
| Found: | 25.26 | 2.60 | 51.36 | 5.57 |

- TLC:: (Silica Gel 60 F254, Merck)
- Eluent:: CHCl3/CH3OH/NH4OH 25% = 3/2/1 v/v/v. Rf = 0.23.
- HPLC:: Rt = 9.6 min. Titre = 99% (on the area)

### Chromatographic conditions:

- Column:: as reported in Example 1
- Eluent:: A) KH2PO4 0.01 M B) H2O 75%, CH3CN 25%

Gradient (flow 1 ml/min; Detector UV 254 nm):

| | | | | |
|---|---|---|---|---|
| time (min) | = | 0 - 5; | 5 - 15; | 15 - 25 |
| %B | = | 20; | 20 - 80; | 80 |

In the same way the following compounds are obtained:
- 1,3-bis-[3-hydroxyacetylamino-5-(2,3-dihydroxypropyl)aminocarbonyl-2,4,6-triiodo-benzoyl-amino]-2,2-bis-hydroxymethyl-propane.
- 1,3-bis-[3-hydroxyacetylamino-5-(1,3,4-trihydroxy-2-butyl)aminocarbonyl-2,4,6-triiodo-benzoylamino]-2,2-bis-hydroxymethyl-propane.

### EXAMPLE 3

### 1,3-bis-[3-(L-2-hydroxy-propionyl)amino-5-(2,3-dihydroxy-propyl)aminocarbonyl-2,4,6-triiodo-benzoyl-amino]-2-hydroxy-propane.

197 g of 3-(L-2-acetoxy-propionyl)amino-5-(2,3-dihydroxy-propyl)aminocarbonyl-2,4,6-triiodo-benzoyl-chloride (0.25 mol), described in DE 2805928/Example 6, are reacted in 500 ml of DMF and in the presence of 55.6 g of tributylamine (0.30 mol) with 11.3 g of 1,3-diamino-2-hydroxy-propane (0.125 mol) according to the procedure described in the Example 2a). The condensation product is submitted to hydrolysis, as described in the Example 2b). Similarly, the isolation and the purification of the final product are performed. 94.2 g of 1,3-bis-[3-(L-2-hydroxy-propionyl)amino-5-(2,3-dihydroxy-propyl)aminocarbonyl-2,4,6-triiodo-benzoyl-amino]-2-hydroxy-propane are obtained.

| | | | |
|---|---|---|---|
| Yield: | 50% | m.p.: | 278-282°C |

| Elemental Analysis (%) | | | | |
|---|---|---|---|---|
| | C | H | I | N |
| Calculated: | 25.47 | 2.48 | 52.09 | 5.75 |
| Found: | 25.21 | 2.53 | 51.89 | 5.59 |

- TLC:: (Silica Gel 60 F₂₅₄, Merck)
- Eluent:: CHCl₃/CH₃OH/NH₄OH 25% = 6/3/1 v/v/v. Rf = 0.24.
- HPLC:: Rt = 15.4 min. Titre = 99.4% (on the area)

### Chromatographic conditions:

- Column:: LICHROSPHER^{R}RP18 (Merck), 5 µ (250 x 4 mm)
- Eluent:: A) H₃PO₄ 0.1 M B) H₂O 50%, CH₃CN 50 %

Gradient (flow 1 ml/min; Detector UV 254 nm):

| | | | | | |
|---|---|---|---|---|---|
| time (min) | = | 0 - 5; | 5 - 20; | 20 - 30; | 30 - 35 |
| %B | = | 10; | 10 - 28; | 28 - 70; | 70 |

### EXAMPLE 4

### 1,3-bis-[3-(L-2-hydroxy-propionyl)amino-5-(1,3,4-tri-hydroxy-2-butyl)aminocarbonyl-2,4,6-triiodo-benzoyl-amino]-2-hydroxy-propane.

24 g of 3-(L-2-acetoxy-propionyl)amino-5-(1,3,4-trihydroxy-2-butyl)aminocarbonyl-2,4,6-triiodo-benzoyl-chloride (0.03 mol), obtained by known methods described in DE 2805928, in 30 ml of DMF and in presence of 3.8 g of triethylamine (0.037 mol) are reacted with a solution of 1.45 g of 1,3-diamino-2-hydroxy-propane (0.016 mol) in 30 ml of DMF at 5 to 8°C, according to the procedure described in Example 3.
7.2 g of the title compound are obtained.

| | | | |
|---|---|---|---|
| Yield: | 30% | m.p.: | 250-256°C |

| Elemental Analysis (%) | | | | |
|---|---|---|---|---|
| | C | H | I | N |
| Calculated: | 26.05 | 2.65 | 50.02 | 5.52 |
| Found: | 26.04 | 2.95 | 49.15 | 5.33 |

[α]²⁰₄₃₆ = -5.01° (c = 10.13% H₂O)
Solubility in H₂0 at 20°C: 100% w/v
- TLC:: (Silica Gel 60 F₂₅₄, Merck)
- Eluent:: CHCl₃/CH₃OH/NH₄OH 25% = 5/4/1 v/v/v. Rf = 0.24.

¹H-and ¹³C-NMR spectra are in accordance with the proposed structure.

### EXAMPLE 5

### 1,3-bis-[3-(L-2-hydroxy-propionyl)amino-5-[N-methyl-N-(D-1-deoxy-glucitol)]aminocarbonyl-2,4,6-triiodo-benzoyl-amino]-2-hydroxy-propane.

20.6 g of 3-(L-2-acetoxy-propionyl)amino-5-[N-methyl-N-(D-1-deoxy-glucitol)]aminocarbonyl-2,4,6-triiodo-benzoyl-chloride (0.024 mol), obtained by known methods described in DE 2805928, in 100 ml of DMF are reacted with a solution of 1.124 g of 1,3-diamino-2-hydroxy-propane (0.012 mol) and 2.53 g of triethylamine (0.025 mol) in 30 ml of DMF, according to the procedure described in Example 3.
9.26 g of the title compound are obtained.

| | | | |
|---|---|---|---|
| Yield: | 47% | m.p.: | 266°C (dec.) |

| Elemental Analysis (%) | | | | |
|---|---|---|---|---|
| | C | H | I | N |
| Calculated: | 28.04 | 3.14 | 45.58 | 5.03 |
| Found: | 28.19 | 3.29 | 45.48 | 5.03 |

[α]²⁰₄₃₆ = -19° (c = 7.04% H₂O)
¹³C-NMR spectrum is in accordance with the proposed structure.
In the same way the following compounds are obtained:
- 1,3-bis-[3-(L-2-hydroxy-propionyl)amino-5-[N-methyl-N-(1,3-dihydroxy-isopropyl)]aminocarbonyl-2,4,6-triiodo-benzoyl-amino]-2-hydroxy-propane.
- 1,3-bis-[3-(L-2-hydroxy-propionyl)amino-5-(N-methyl-N-(2,3-dihydroxy-propyl)]aminocarbonyl-2,4,6-triiodo-benzoyl-amino]-2-hydroxy-propane.

### EXAMPLE 6

### 1,3-bis-[N-methyl-N-[3-(L-2-hydroxy-propionyl)amino-5-(2-hydroxy-ethyl)aminocarbonyl-2,4,6-triiodo-benzoyl]-amino]-2-hydroxy-propane.

20.85 g of 3-(L-2-acetoxy-propionyl)amino-5-(2-hydroxy-ethyl)aminocarbonyl-2,4,6-triiodo-benzoyl-chloride (0.025 mol), obtained by known methods described in DE 2805928, in 80 ml of DMF are reacted with a solution of 1.47 g of 1,3-bis-(methylamino)-2-hydroxy-propane (0.012 mol) and 2.63 g of triethylamine (0.026 mol) in 30 ml of DMF at 5°C to room temperature, according to the procedure described in Example 3.
9.6 g of the title compound are obtained.

| | | | |
|---|---|---|---|
| Yield: | 56% | m.p.: | 285°C (dec.) |

| Elemental Analysis (%) | | | | |
|---|---|---|---|---|
| | C | H | I | N |
| Calculated: | 26.03 | 2.54 | 53.24 | 5.88 |
| Found: | 25.86 | 2.43 | 52.81 | 5.78 |

[α]²⁰₄₃₆ = -3.85° (c = 9.95% H₂O)
¹H-and ¹³C-NMR spectra are in accordance with the proposed structure.

### EXAMPLE 7

### 1,3-bis-[N-methyl-N-[3-(L-2-hydroxy-propionyl)amino-5-(1,3-dihydroxy-isopropyl)aminocarbonyl-2,4,6-triiodo-benzoyl]amino]-2-hydroxy-propane.

21.4 g of 3-(L-2-acetoxy-propionyl)amino-5-(1,3-dihydroxy-isopropyl)aminocarbonyl-2,4,6-triiodo-benzoylchloride (0.025 mol), obtained by known methods described in DE 2805928, in 85 ml of DMF are reacted with a solution of 1.47 g of 1,3-bis-(methylamino)-2-hydroxy-propane (0.012 mol) and 2.63 g of triethylamine (0.026 mol) in 30 ml of DMF at 5°C to room temperature, according to the procedure described in Example 3.
11.1 g of the title compound are obtained.

| | | | |
|---|---|---|---|
| Yield: | 62% | m.p.: | 295°C (dec.) |

| Elemental Analysis (%) | | | | |
|---|---|---|---|---|
| | C | H | I | N |
| Calculated: | 26.60 | 2.70 | 51.09 | 5.64 |
| Found: | 26.31 | 2.95 | 50.68 | 5.48 |

[α]²⁰₄₃₆ = -3.54° (c = 10.44% H₂O)
Solubility in H₂O at 22°C: > 100% w/v
¹H-and ¹³C-NMR spectra are in accordance with the proposed structure.

### EXAMPLE 8

### 1,3-bis-[N-methyl-N-[3-(L-2-hydroxy-propionyl)amino-5-(2,3-dihydroxy-propyl)aminocarbonyl-2,4,6-triiodobenzoyl]amino]-2-hydroxy-propane.

Title compound is obtained by reacting the desired reagents in the same quantities disclosed in Example 7 and following the same procedure. 7.9 g of final compound are obtained.

| | | | |
|---|---|---|---|
| Yield: | 44% | m.p.: | 280°C (dec.) |

| Elemental Analysis (%) | | | | |
|---|---|---|---|---|
| | C | H | I | N |
| Calc.: | 26.60 | 2.70 | 51.09 | 5.64 |
| Found: | 26.32 | 2.81 | 50.24 | 5.44 (H₂O=1.54%) |

[α]²⁰₄₃₆ = -3.2° (c = 9.8% H₂O)
Solubility in H₂0 at 20°C: >100% w/v
¹³C-NMR spectrum is in accordance with the proposed structure.

### EXAMPLE 9

### 1,3-bis-[N-methyl-N-[3-(L-2-hydroxy-propionyl)amino-5-(1,3,4-trihydroxy-2-butyl)aminocarbonyl-2,4,6-triiodo-benzoyl]amino]-2-hydroxy-propane.

19.9 g of 3-(L-2-acetoxy-propionyl)amino-5-(1,3,4-trihydroxy-2-butyl)aminocarbonyl-2,4,6-triiodo-benzoyl-chloride (0.025 mol), obtained by known methods described in DE 2805928, in 30 ml of DMF are mixed with 3.8 g of triethylamine (0.037 mol), then are reacted with a solution of 1.5 g of 1,3-bis-(methylamino)-2-hydroxy-propane (0.013 mol) in 25 ml of DMF at 5° C to room temperature, according to the procedure described in Example 3.
5.68 g of the title compound are obtained.

| | | | |
|---|---|---|---|
| Yield: | 29.5% | m.p.: | 256°C |

| Elemental Analysis (%) | | | | |
|---|---|---|---|---|
| | C | H | I | N |
| Calculated: | 27.12 | 2.86 | 49.12 | 5.42 |
| Found: | 27.46 | 3.15 | 48.55 | 5.24 |

[α]²⁰₄₃₆ = -3.16° (c = 9.93% H₂O)
¹H-and ¹³C-NMR spectra are in accordance with the proposed structure.

### EXAMPLE 10

### 1-[3-(L-2-hydroxy-propionyl)amino-5-(1,3-dihydroxyisopropyl)aminocarbonyl-2,4,6-triiodo-benzoyl-amino]-3-[3-hydroxyacetylamino-5-(2,3-dihydroxy-propyl)aminocarbonyl-2,4,6-triiodo-benzoyl-amino]-2-hydroxy-propane.

a) 125 g of 3-(L-2-acetoxy-propionyl)amino-5-(1,3-dihydroxy-isopropyl)aminocarbonyl-2,4,6-triiodobenzoyl-chloride (0.163 mol), diluted in 150 ml of DMF, are dropwise added, while keeping the temperature between 0 and 10°C, to a solution of 45.6 g of 1-trifluoroacetylamino-2-hydroxy-3-aminopropane trifluoroacetate (0.152 mol) and 35.8 g of triethylamine (0.35 mol) in 280 ml of DMF. The resulting mixture is stirred at 10°C for 8 h. After filtration, the solution is evaporated to dryness, then is diluted with 3 l of methylene chloride (CH₂Cl₂). The condensation product precipitates, then, after filtration, the protective groups are removed by hydrolysis, according to the procedure described in Example 2b). The resulting product is purified by means of fixation on a strongly acidic resin (950 ml of Amberlite^{R} IR 120) and subsequent elution with about 6 l of NH₄OH 2M. After evaporation of the solvent, 76.5 g of 1-[3-(L-2-hydroxy-propionyl)amino-5-(1,3-dihydroxyisopropyl)aminocarbonyl-2,4,6-triiodo-benzoylamino]-2-hydroxy-3-amino-propane are obtained with a yield of 65%.

| | | | |
|---|---|---|---|
| Acidimetric titre = | 99.4% | m.p.: | 211°C |

b) 73.5 g of 3-acetoxyacetylamino-5-(2,3-dihydroxypropyl)aminocarbonyl-2,4,6-triiodo-benzoyl-chloride (0.098 mol) in 500 ml of DMF are slowly added dropwise, at a temperature between 5 and 10°C, to a solution of 76 g of the product obtained at point a) (0.098 mol) and 11.1 g of triethylamine (0.11 mol) in 500 ml of DMF. The mixture is kept under stirring for 20 h, then filtered and dried. The residue is dissolved in 1.9 l of water and 0.8 l of methyl alcohol, then it is submitted to hydrolysis and purification according to the procedure described in Example 2b). 92 g of 1-[3-(L-2-hydroxy-propionyl)amino-5-(1,3-dihydroxy-isopropyl)aminocarbonyl-2,4,6-triiodo-benzoyl-amino]-3-[3-hydroxyacetylamino-5-(2,3-dihydroxypropyl)aminocarbonyl-2,4,6-triiodo-benzoyl-amino]-2-hydroxypropane are obtained.

| | | | |
|---|---|---|---|
| Yield: | 64.8% | m.p.: | 300°C |

| Elemental Analysis (%) | | | | |
|---|---|---|---|---|
| | C | H | I | N |
| Calculated: | 24.88 | 2.37 | 52.58 | 5.80 |
| Found: | 24.70 | 2.29 | 52.15 | 5.74 |

[α]²⁰₄₃₆ = -2.9° (c = 10% H₂O)
- TLC:: (Silica Gel 60 F₂₅₄, Merck)
- Eluent:: CHCl₃/CH₃OH/NH₄OH 25% = 6/3/1 v/v/v. Rf = 0.18.
- HPLC:: Rt = 13.6 min. Titre = 97.5% (on the area)

### Chromatographic conditions:

- Column:: as reported in Example 1
- Eluents:: A) K₂PO₄ 0.01 M B) KH₂PO₄ 0.01M 50%, CH₃CN 50%

Gradient (flow 1 ml/min; Detector UV 254 nm):

| | | | | |
|---|---|---|---|---|
| time (min) | = | 0 - 6; | 6 - 25; | 25 - 30 |
| %B | = | 2; | 2 - 80; | 80 |

### EXAMPLE 11

### 1-[3-hydroxyacetylamino-5-(1,3,4-trihydroxy-2-butyl)aminocarbonyl-2,4,6-triiodo-benzoyl-amino]-3-[3-(L-2-hydroxy-propionyl)amino-5-(1,3-dihydroxy-isopropyl)aminocarbonyl-2,4,6-triiodo-benzoyl-amino]-2-hydroxypropane.

Title compound is obtained following the procedure described in Example 10.

So 8.58 g of 3-acetoxyacetylamino-5-(1,3,4-trihydroxy-2-butyl)aminocarbonyl-2,4,6-triiodo-benzoyl-chloride (0.011 mol) in 60 ml of DMF are reacted with a solution of 8.54 g 1-[3-(L-2-hydroxy-propionyl)amino-5-(1,3-dihydroxy-isopropyl)aminocarbonyl-2,4,6-triiodo-benzoyl-amino]-2-hydroxy-3-amino-propane (0.011 mol), obtained according to Example 10 a), and 2.22 g of triethylamine (0.022 mol) in 60 ml of DMF to give the desired compound.
8.75 g of the title compound are obtained.

| | | | |
|---|---|---|---|
| Yield: | 51% | m.p.: | 283°C (dec.) |

| Elemental Analysis (%) | | | | |
|---|---|---|---|---|
| | C | H | I | N |
| Calc.: | 25.19 | 2.45 | 51.51 | 5.68 |
| Found: | 24.13 | 2.72 | 48.88 | 5.32 (H₂O = 2.71%) |

[α]²⁰₄₃₆ = -2.6° (c = 10.44% H₂O)
Solubility in H₂0 at 25°C: >100% w/v
¹³C-NMR spectrum is in accordance with the proposed structure.

### EXAMPLE 12

### 1-[3-(L-2-hydroxy-propionyl)amino-5-(1,3-dihydroxyisopropyl)aminocarbonyl-2,4,6-triiodo-benzoyl-amino]-3-[3-(L-2-hydroxy-propionyl)amino-5-(2,3-dihydroxypropyl)-aminocarbonyl-2,4,6-triiodo-benzoyl-amino]-2-hydroxy-propane.

Following the procedure described in Example 10, 8.75 g of 3-(L-2-acetoxy-propionyl)amino-5-(2,3-dihydroxy-propyl)aminocarbonyl-2,4,6-triiodo-benzoyl-chloride (0.01 mol) in 60 ml of DMF are reacted with a solution of 7.76 g 1-[3-(L-2-hydroxy-propionyl)amino-5-(1,3-dihydroxy-isopropyl)aminocarbonyl-2,4,6-triiodo-benzoyl-amino]-2-hydroxy-3-amino-propane (0.01 mol), obtained according to Example 10 a), and 2.02 g of triethylamine (0.02 mol) in 60 ml of DMF to give the desired compound. 8.15 g of the title compound are obtained.

| | | | |
|---|---|---|---|
| Yield: | 55% | m.p.: | 287°C (dec.) |

| Elemental Analysis (%) | | | | |
|---|---|---|---|---|
| | C | H | I | N |
| Calculated: | 25.46 | 2.48 | 52.08 | 5.75 |
| Found: | 25.49 | 2.34 | 51.67 | 5.73 |

[α]²⁰₄₃₆ = -5.67° (c = 10.01% H₂O)
Solubility in H₂0 at 20°C: >100% w/v
¹³C-NMR spectrum is in accordance with the proposed structure.

### EXAMPLE 13

### 1-[3-(L-2-hydroxy-propionyl)amino-5-(1,3-dihydroxyisopropyl)aminocarbonyl-2,4,6-triiodo-benzoyl-amino]-3-[3-(L-2-hydroxy-propionyl)amino-5-[N-methyl-N-(D-1-deoxy-glucitol)]aminocarbonyl-2,4,6-triiodo-benzoyl-amino]-2-hydroxy-propane.

Following the procedure described in Example 10, 6.9 g of 3-(L-2-acetoxy-propionyl)-5-[N-methyl-N-(D-1-deoxy-glucitol)]aminocarbonyl-2,4,6-triiodo-benzoyl-chloride (0.008 mol) in 50 ml of DMF are reacted with a solution of 6.13 g 1-[(L-2-hydroxy-propionyl)-5-(1,3-dihydroxy-isopropyl)aminocarbonyl-2,4,6-triiodo-benzoyl-amino]-2-hydroxy-3-amino-propane (0.008 mol), obtained according to Example 10 a), and 1.6 g of triethylamine (0.016 mol) in 50 ml of DMF to give the desired compound.
4.2 g of the title compound are obtained.

| | | | |
|---|---|---|---|
| Yield: | 34% | m.p.: | 285°C (dec.) |

| Elemental Analysis (%) | | | | |
|---|---|---|---|---|
| | C | H | I | N |
| Calculated: | 26.84 | 2.83 | 48.62 | 5.37 |
| Found: | 27.29 | 2.92 | 48.62 | 5.22 |

[α]²⁰₄₃₆ = -11.9° (c = 5.0% H₂O)
Solubility in H₂0 at 20°C: >100% w/v
¹³C-NMR spectrum is in accordance with the proposed structure.

### EXAMPLE 14

### 1,3-bis-[3-hydroxyacetylamino-5-(1,3-dihydroxy-isopropyl)aminocarbonyl-2,4,6-triiodo-benzoyl-amino]-2-hydroxy-propane.

112.5 g of 3-acetoxyacetylamino-5-(1,3-dihydroxy-isopropyl)aminocarbonyl-2,4,6-triiodo-benzoy1-chloride (0.15 mol), described in DE 2805928/Example 10, in 400 ml of DMF are reacted with 16.7 g of triethylamine (0.165 mol) and with 7.13 g of 1,3-diamino-2-hydroxy-propane (0.075 mol), according to the procedure described in Example 2.

63 g of 1,3-bis-[3-hydroxyacetylamino-5-(1,3-dihydroxy-isopropyl)aminocarbonyl-2,4,6-triiodo-benzoyl-amino]-2-hydroxy-propane are obtained.

| | | | |
|---|---|---|---|
| Yield: | 56% | m.p.: | > 280°C |

| Elemental Analysis (%) | | | | |
|---|---|---|---|---|
| | C | H | I | N |
| Calculated: | 24.49 | 2.25 | 53.10 | 5.86 |
| Found: | 24.19 | 2.28 | 53.26 | 5.83 |

- TLC:: (Silica Gel 60 F₂₅₄, Merck)
- Eluent:: CHCl₃/CH₃OH/NH₄OH 25% = 3/2/1 v/v. Rf = 0.60.
- HPLC:: Rt = 7.1 min. Titre = 99% (on the area)

Chromatographic conditions: as reported in Example 2.

### EXAMPLE 15

### 1,3-bis-[3-hydroxyacetylamino-5-(1,3,4-trihydroxy-2-butyl)aminocarbonyl-2,4,6-triiodo-benzoyl-amino]-2-hydroxy-propane.

23.4 g of 3-acetoxyacetylamino-5-(1,3,4-trihydroxy-2-butyl)aminocarbonyl-2,4,6-triiodo-benzoyl-chloride (0.03 mol), obtained by known methods described in DE 2805928, in 25 ml of DMF are reacted with 3.8 g of triethylamine (0.037 mol) and with 1.45 g of 1,3-diamino-2-hydroxy-propane (0.016 mol), according to the procedure described in Example 2.
4.7 g of the title compound are obtained.

| | | | |
|---|---|---|---|
| Yield: | 21% | m.p.: | 275°C |

| Elemental Analysis (%) | | | | |
|---|---|---|---|---|
| | C | H | I | N |
| Calculated: | 24.92 | 2.43 | 50.96 | 5.62 |
| Found: | 24.93 | 2.50 | 49.98 | 5.53 |

Solubility in H₂0 at 20°C: 100% w/v
In the same way the following compounds are obtained:
- 1,3-bis-[3-hydroxyacetylamino-5-(2,3-dihydroxypropyl)aminocarbonyl-2,4,6-triiodo-benzoyl-amino]-2-hydroxy-propane.
- 1,3-bis-[3-hydroxyacetylamino-5-[N-methyl-N-(1,3-dihydroxy-isopropyl)]aminocarbonyl-2,4,6-triiodo-benzoyl-amino]-2-hydroxy-propane.
- 1,3-bis-[3-hydroxyacetylamino-5-[N-methyl-N-(2,3-dihydroxy-propyl)]aminocarbonyl-2,4,6-triiodo-benzoyl-amino]-2-hydroxy-propane.

### EXAMPLE 16

### 1,3-bis-[N-methyl-N-[3-hydroxyacetylamino-5-(1,3,4-trihydroxy-2-butyl)aminocarbonyl-2,4,6-triiodo-benzoyl]-amino]-2-hydroxy-propane.

23.4 g of 3-acetoxyacetylamino-5-(1,3,4-trihydroxy-2-butyl)aminocarbonyl-2,4,6-triiodo-benzoyl-chloride (0.03 mol), in 25 ml of DMF are reacted with 3.8 g of triethylamine (0.037 mol) and with 1.9 g of 1,3-bis-(methylamino)-2-hydroxy-propane (0.016 mol), according to the procedure described in Example 2.
7 g of the title compound are obtained.

| | | | |
|---|---|---|---|
| Yield: | 30% | m.p.: | 263°C |

| Elemental Analysis (%) | | | | |
|---|---|---|---|---|
| | C | H | I | N |
| Calculated: | 26.04 | 2.65 | 50.02 | 5.52 |
| Found: | 26.12 | 2.69 | 50.86 | 5.57 |

Solubility in H₂0 at 20°C: 100% w/v

### EXAMPLE 17

### 1,3-bis-[N-methyl-N-[3-hydroxyacetylamino-5-[N-methyl-N-(D-1-deoxy-glucitol)]aminocarbonyl-2,4,6-triiodo-benzoyl]amino]-2-hydroxy-propane.

24 g of 3-acetoxyacetylamino-5-[N-methyl-N-(D-1-deoxy-glucitol)]aminocarbonyl-2,4,6-triiodo-benzoyl-chloride (0.028 mol), obtained by known methods described in DE 2805928, in 100 ml of DMF are reacted with a solution of 1.65 g of 1,3-bis-(methylamino)-2-hydroxy-propane (0.014 mol) and 2.93 g of triethylamine (0.029 mol) in 30 ml of DMF, according to the procedure described in Example 2.
10.14 g of the title compound are obtained.

| | | | |
|---|---|---|---|
| Yield: | 41% | m.p.: | 276°C (dec.) |

| Elemental Analysis (%) | | | | |
|---|---|---|---|---|
| | C | H | I | N |
| Calculated: | 28.04 | 3.14 | 45.58 | 5.03 |
| Found: | 27.95 | 3.21 | 45.48 | 4.97 |

[α]²⁰₄₃₆ = -12.6° (c = 10.6% H₂O)
Solubility in H₂0 at 25°C: >100% w/v
¹³C-NMR spectrum is in accordance with the proposed structure.
In the same way the following compounds are obtained:
- 1,3-bis-[N-methyl-N-[3-hydroxyacetylamino-5-(1,3-dihydroxy-isopropyl)aminocarbonyl-2,4,6-triiodo-benzoyl]amino]-2-hydroxypropane.
- 1,3-bis-[N-methyl-N-[3-hydroxyacetylamino-5-(2,3-dihydroxy-propyl)aminocarbonyl-2,4,6-triiodo-benzoyl]amino]-2-hydroxy-propane.

### EXAMPLE 18

### 1,3-bis-[N-methyl-N-[3-hydroxyacetylamino-5-[N-methyl-N-(1,3-dihydroxy-isopropyl)]aminocarbonyl-2,4,6-triiodo-benzoyl]amino]-2-hydroxy-propane.

21.31 g of 3-acetoxyacetylamino-5-[N-methyl-N-(1,3-dihydroxy-isopropyl)]aminocarbonyl-2,4,6-triiodo-benzoyl-chloride (0.025 mol), obtained by known methods described in DE 2805928, in 95 ml of DMF are reacted with a solution of 1.47 g of 1,3-bis-(methylamino)-2-hydroxy-propane (0.12 mol) and 2.63 g of triethylamine (0.026 mol) in 30 ml of DMF, according to the procedure described in Example 2.
8.1 g of the title compound are obtained.

| | | | |
|---|---|---|---|
| Yield: | 45% | m.p.: | 290°C |

| Elemental Analysis (%) | | | | |
|---|---|---|---|---|
| | C | H | I | N |
| Calc.: | 26.98 | 2.86 | 50.33 | 5.55 |
| Found: | 26.29 | 3.01 | 50.21 | 5.24 (H₂O = 1.02%) |

Solubility in H₂0 at 25°C: 100% w/v
¹³C-NMR spectrum is in accordance with the proposed structure.

### EXAMPLE 19

### 1,3-bis-[3-(N-methyl-N-hydroxyacetyl)amino-5-(1,3-dihydroxy-isopropyl)aminocarbonyl-2,4,6-triiodo-benzoyl-amino]-2-hydroxy-propane.

a) 46 g of 3-(N-methyl-N-acetoxyacetyl)amino-2,4,6-triiodo-isophthaloyl-dichloride (0.065 mol), described in EP 26281/Example 11, in 250 ml of anhydrous tetrahydrofuran (THF) are added dropwise to 12.6 g of 1,3-dihydroxy-isopropylamine (0.138 mol) in 100 ml of THF. The mixture is kept for 3 h at room temperature under stirring. Then it is filtered and evaporated to dryness under vacuum. The residue is constituted by 50.4 g of 3-(N-methyl-N-acetoxyacetyl)amino-5-(1,3-dihydroxy-isopropyl)aminocarbonyl-2,4,6-triiodo-benzoyl-chloride.
b) 50 g of 3-(N-methyl-N-acetoxyacetyl)amino-5-(1,3-dihydroxy-isopropyl)aminocarbonyl-2,4,6-triiodo-benzoyl-chloride (about 0.065 mol), obtained according to a), diluted in 200 ml of DMF, are reacted with 3 g of 1,3-diamino-2-hydroxy-propane (0.034 mol) in 100 ml of DMF, according to the procedure described in Example 1.

21 g of 1,3-bis-[3-(N-methyl-N-hydroxyacetyl)amino-5-(1,3-dihydroxy-isopropyl)aminocarbonyl-2,4,6-triiodo-benzoyl-amino]-2-hydroxy-propane are obtained.

| | | | |
|---|---|---|---|
| Yield: | 44.2% | m.p.: | > 250°C |

| Elemental Analysis (%) | | | | |
|---|---|---|---|---|
| | C | H | I | N |
| Calculated: | 25.47 | 2.48 | 52.08 | 5.75 |
| Found: | 25.82 | 2.60 | 52.37 | 5.62 |

- TLC:: (Silica Gel 60 F₂₅₄, Merck)
- Eluent:: CHCl₃/CH₃OH/NH₄OH 25% = 6/3/1 v/v/v. Rf = 0.23
- HPLC:: Rt = 14.6 min. Titre = 96.7% (on the area)

Chromatographic conditions: as reported in Example 2.
c) This product may be also obtained by N-methylation of the compound already described in Example 14. 14.3 g of 1,3-bis-[3-hydroxyacetylamino-5-(1,3-dihydroxy-isopropyl)aminocarbonyl-2,4,6-triiodo-benzoyl-amino]-2-hydroxy-propane (0.01 mol) in 500 ml of anhydrous DMF are mixed under stirring with a solution of 1.08 g of sodium methylate (0.02 mol) in 30 ml of methyl alcohol. After 3 h, methyl alcohol is removed by distillation. An excess of methyl iodide is added and the solution is stirred for one day at room temperature. Then the reaction mixture is poured under stirring in 600 ml of ethyl acetate and the raw product precipitates. This one is filtered, diluted in water and made free from salts by passage through ion exchange resins (Amberlite^{R} IR 120 and IRA 400). The eluate is treated with active carbon and evaporated to dryness.

7 g of 1,3-bis-[3-(N-methyl-N-hydroxyacetyl)amino-5-(1,3-dihydroxy-isopropyl)aminocarbonyl-2,4,6-triiodo-benzoyl-amino]-2-hydroxy-propane are obtained with a yield of 47.9%. This compound is identical to the one obtained through process b).

In the same way the following compound is obtained:
- 1,3-bis-[3-[N-methyl-N-(L-2-hydroxy-propionyl)]-amino-5-(1,3,4-trihydroxy-2-butyl)aminocarbonyl-2,4,6-triiodo-benzoyl-amino]-2-hydroxy-propane.

### EXAMPLE 20

### 1,3-bis-[3-[N-methyl-N-(L-2-hydroxy-propionyl)]amino-5-(1,3-dihydroxy-isopropyl)aminocarbonyl-2,4,6-triiodo-benzoyl-amino]-2-hydroxy-propane.

80.4 g of 1,3-bis-[3-(L-2-hydroxy-propionyl)amino-5-(1,3-dihydroxy-isopropyl)aminocarbonyl-2,4,6-triiodo-benzoyl-amino]-2-hydroxy-propane (0.055 mol), described in Example 1, are dissolved in 900 ml of DMF and mixed under stirring with 132 ml of sodium methylate 1 M in methyl alcohol (0.132 mol). Methyl alcohol is removed by distillation. Hence, 23.7 g of dimethylsulphate (0.188 mol) are dropwise added at a temperature of 5°C. Stirring is maintained for about 1 h, then the solvent is removed by vacuum distillation. The raw material is diluted with ethyl acetate and forms a solid powder, which is filtered, diluted in water and made free from salts by percolation on ion exchange resins (Amberlite^{R} IR 120 and Duolite^{R} A 30B). The solution is evaporated to dryness and then dissolved in boiling ethyl alcohol. After some days, 62 g of 1,3-bis-[3-[N-methyl-N-(L-2-hydroxy-propionyl)]amino-5-(1,3-dihydroxy-isopropyl)-aminocarbonyl-2,4,6-triiodo-benzoyl-amino]-2-hydroxy-propane crystallize.

| | | | |
|---|---|---|---|
| Yield: | 75.6% | m.p.: | > 300°C (dec.) |

| Elemental Analysis (%) | | | | |
|---|---|---|---|---|
| | C | H | I | N |
| Calculated: | 26.60 | 2.70 | 51.10 | 5.64 |
| Found: | 26.58 | 2.75 | 50.95 | 5.60 |

[α]²⁰₄₃₆ = +29.7° (c = 10% H₂O)
- TLC:: (Silica Gel 60 F₂₅₄, Merck)
- Eluent:: CHCl₃/CH₃OH/NH₄OH 25% = 6/3/1 v/v/v. Rf = 0.16.

### EXAMPLE 21

### 1,3-bis-[3-[N-methyl-N-(L-2-hydroxy-propionyl)]amino-5-(2,3-dihydroxy-propyl)aminocarbonyl-2,4,6-triiodobenzoyl-amino]-2-hydroxy-propane.

In 250 ml of DMF, 102 g of the product described in Example 3 (0.07 mol) are reacted with 150 ml of sodium methylate 1M in methyl alcohol (0.15 mol) and with 30 g of metansulphonate methyl ester (0.27 mol), according to the procedure described in Example 20.

42.6 g of 1,3-bis-[3-[N-methyl-N-(L-2-hydroxy-propionyl)]amino-5-(2,3-dihydroxy-propyl)aminocarbonyl-2,4,6-triiodo-benzoyl-amino]-2-hydroxy-propane are obtained.

| | | | |
|---|---|---|---|
| Yield: | 41% | m.p.: | 284°C |

| Elemental Analysis (%) | | | | |
|---|---|---|---|---|
| | C | H | I | N |
| Calculated: | 26.50 | 2.70 | 51.10 | 5.64 |
| Found: | 26.24 | 2.80 | 50.88 | 5.47 |

### EXAMPLE 22

### 1,3-bis-[3-(N-methyl-N-hydroxyacetyl)amino-5-(2,3-dihydroxy-propyl)aminocarbonyl-2,4,6-triiodo-benzoyl-amino]-2-hydroxy-propane.

23 g of 3-(N-methyl-N-acetoxyacetyl)amino-2,4,6-triiodo-isophthaloyl-dichloride (0.032 mol), described in EP 26281/Example 11, are reacted with 5.8 g of 2,3-dihydroxy-propylamine (0.064 mol) in THF according to the procedure described in Example 19a). The resulting product is 3-(N-methyl-N-acetoxyacetyl)amino-5-(2,3-dihydroxy-propyl)aminocarbonyl-2,4,6-triiodo-benzoyl-chloride, which is reacted in DMF with 1.5 g of 1,3-diamino-2-hydroxy-propane (0.016 mol). The resulting product is isolated and purified according to the procedure of Example 1. 11 g of 1,3-bis-[3-(N-methyl-N-hydroxyacetyl)amino-5-(2,3-dihydroxy-propyl)aminocarbonyl-2,4,6-triiodo-benzoyl-amino]-2-hydroxy-propane are obtained.

| | | | |
|---|---|---|---|
| Yield: | 47% | m.p.: | 295°C (dec.) |

| Elemental Analysis (%) | | | | |
|---|---|---|---|---|
| | C | H | I | N |
| Calculated: | 25.47 | 2.48 | 52.08 | 5.75 |
| Found: | 25.97 | 2.50 | 51.71 | 5.77 |

In the same way the following compound is obtained:
- 1,3-bis-[3-(N-methyl-N-hydroxyacetyl)amino-5-(1,3,4-trihydroxy-2-butyl)]aminocarbonyl-2,4,6-triiodo-benzoyl-amino]-2-hydroxy-propane.

### EXAMPLE 23

### 1,3-bis-[3-(L-2-hydroxy-propionyl)amino-5-(1,3-dihydroxyisopropyl)aminocarbonyl-2,4,6-triiodo-benzoyl-amino]-2,2-bis-hydroxymethyl-propane.

Following the procedure described in Example 2, the 3-(L-2-acetoxy-propionyl)amino-5-(1,3-dihydroxy-isopropyl)aminocarbonyl-2,4,6-triiodo-benzoyl-chloride is reacted in DMF with 1,3-diamino-2,2-bis-hydroxymethyl-propane to give the desired product.

| | | | |
|---|---|---|---|
| Yield: | 65% | m.p.: | 285°C (dec.) |

| Elemental Analysis (%) | | | | |
|---|---|---|---|---|
| | C | H | I | N |
| Calculated: | 26.32 | 2.68 | 50.55 | 5.58 |
| Found: | 26.21 | 2.72 | 50.31 | 5.53 |

- TLC:: (Silica Gel 60 F₂₅₄, Merck)
- Eluent:: CHCl₃/CH₃OH/NH₄OH 25% = 6/3/1 v/v/v. Rf = 0.12
- HPLC:: Rt = 17.7 min. Titre = 98% (on the area)

Chromatographic conditions: as reported in Example 3.

### EXAMPLE 24

### 1,3-bis-[3-(L-2-hydroxy-propionyl)amino-5-[N-methyl-N-(D-1-deoxy-glucitol)]aminocarbonyl-2,4,6-triiodo-benzoyl-amino]-2,2-bis-hydroxymethyl-propane.

Following the procedure described in Example 2, 10 g of 3-(L-2-acetoxy-propionyl)amino-5-[N-methyl-N-(D-1-deoxy-glucitol)]aminocarbonyl-2,4,6-triiodo-benzoyl-chloride (0.011 mol) in 50 ml of DMF are reacted with 1.19 g of 1,3-diamino-2,2-bis-hydroxymethyl-propane (0.006 mol) and 4.94 g of tributylamine (0.027 mol) in 20 ml of DMF.
11.5 g of the title compound are obtained.

| | |
|---|---|
| Yield: | 58% |

| Elemental Analysis (%) | | | | |
|---|---|---|---|---|
| | C | H | I | N |
| Calculated: | 28.72 | 3.29 | 44.41 | 4.90 |
| Found: | 28.60 | 3.33 | 44.37 | 4.85 |

¹³C-NMR spectrum is in accordance with the proposed structure.
In the same way the following compounds are obtained:
- 1,3-bis-[3-(L-2-hydroxy-propionyl)amino-5-(2,3-dihydroxy-propyl)aminocarbonyl-2,4,6-triiodo-benzoyl-amino)-2,2-bis-hydroxymethyl-propane.
- 1,3-bis(3-(L-2-hydroxy-propionyl)amino-5-(1,3,4-trihydroxy-2-butyl)aminocarbonyl-2,4,6-triiodo-benzoyl-amino]-2,2-bis-hydroxymethyl-propane.

### EXAMPLE 25

### 1,3-bis-[3-[N-methyl-N-(L-2-hydroxy-propionyl)]amino-5-(1,3-dihydroxy-isopropyl)aminocarbonyl-2,4,6-triiodo-benzoyl-amino]-2,2-bis-hydroxymethyl-propane.

The product described in Example 23 is submitted to N-methylation with sodium methylate and CH₃I following the procedure of Example 19c).

| | | | |
|---|---|---|---|
| Yield: | 65.5% | m.p.: | > 300°C |

| Elemental Analysis (%) | | | | |
|---|---|---|---|---|
| | C | H | I | N |
| Calculated: | 27.40 | 2.89 | 49.63 | 5.48 |
| Found: | 27.38 | 2.91 | 49.47 | 5.45 |

[α]²⁰₄₃₆ = +26.7° (c = 10% H₂O)
In the same way the following compounds are obtained:
- 1,3-bis-[3-[N-methyl-N-(L-2-hydroxy-propionyl)]-amino-5-(2,3-dihydroxy-propyl)aminocarbonyl-2,4,6-triiodo-benzoyl-amino]-2,2-bis-hydroxymethyl-propane.
- 1,3-bis-[3-[N-methyl-N-(L-2-hydroxy-propionyl)]-amino-5-(1,3,4-trihydroxy-propyl)aminocarbonyl-2,4,6-triiodo-benzoyl-amino]-2,2-bis-hydroxymethyl-propane.

### EXAMPLE 26

### 1,3-bis-[N-methyl-N-[3-[N-methyl-N-(L-2-hydroxy-propionyl)]amino-5-(2-hydroxy-ethyl)aminocarbonyl-2,4,6-triiodo-benzoyl]amino]-2-hydroxy-propane.

The product described in Example 6 is submitted to N-methylation with sodium methylate and CH₃I following the procedure of Example 19 c).

| | | | |
|---|---|---|---|
| Yield: | 58% | m.p.: | 280°C |

| Elemental Analysis (%) | | | | |
|---|---|---|---|---|
| | C | H | I | N |
| Calculated: | 27.18 | 2.76 | 52.22 | 5.76 |
| Found: | 27.25 | 2.77 | 52.14 | 5.91 |

¹³C-NMR spectrum is in accordance with the proposed structure.

### EXAMPLE 27

### 1,3-bis-[N-methyl-N-[3-[N-methyl-N-(L-2-hydroxy-propionyl)]amino-5-(1,3-dihydroxy-isopropyl)aminocarbonyl-2,4,6-triiodo-benzoyl]amino]-2-hydroxy-propane.

The product described in Example 7 is submitted to N-methylation with sodium methylate and CH₃I following the procedure of Example 19 c).

| | | | |
|---|---|---|---|
| Yield: | 54% | m.p.: | 285°C (dec.) |

| Elemental Analysis (%) | | | | |
|---|---|---|---|---|
| | C | H | I | N |
| Calc.: | 27.69 | 2.92 | 50.15 | 5.53 |
| Found: | 25.25 | 3.23 | 48.72 | 5.27 (H2O = 2.7%) |

Solubility in H2O at 20°C: 100% w/v
13C-NMR spectrum is in accordance with the proposed structure.

### EXAMPLE 28

### 1,3-bis-[3-(N-methyl-N-hydroxyacetyl)amino-5-(1,3-dihydroxy-isopropyl)aminocarbonyl-2,4,6-triiodo-benzoyl-amino]-2,2-bis-hydroxymethyl-propane.

The product described in Example 2 is reacted with 2 equivalent of NaOH, hence with an excess of CH₃Br in DMF for one day under stirring in a sealed system. The subsequent work follows the procedure described in Example 19 c).

| | | | |
|---|---|---|---|
| Yield: | 54% | m.p.: | > 285-288°C |

| Elemental Analysis (%) | | | | |
|---|---|---|---|---|
| | C | H | I | N |
| Calculated: | 26.32 | 2.68 | 50.55 | 5.58 |
| Found: | 25.90 | 2.80 | 50.12 | 5.53 |

- TLC:: (Silica Gel 60 F₂₅₄, Merck)
- Eluent:: CHCl₃/CH₃OH/NH₄OH 25% = 3/2/1 v/v/v. Rf = 0.26
- HPLC:: Rt = 15.5 min. Titre = 97.7% (on the area)

Chromatographic conditions: as reported in Example 2.
In the same way the following compounds are obtained:
- 1,3-bis-[3-(N-methyl-N-hydroxyacetyl)amino-5-(2,3-dihydroxy-propyl)aminocarbonyl-2,4,6-triiodo-benzoyl-amino]-2,2-bis-hydroxymethyl-propane.
- 1,3-bis-[3-(N-methyl-N-hydroxyacetyl)amino-5-(1,3,4-trihydroxy-2-butyl)aminocarbonyl-2,4,6- triiodo-benzoyl-amino]-2,2-bis-hydroxymethylpropane.

**Table 1**

| Acute toxicity of an aqueous solution of 1,3-bis[3-(L-2-hydroxy-propionyl)amino-5-(1,3-dihydroxyisopropyl)aminocarbonyl-2,4,6-triiodo-benzoyl-amino]-2-hydroxy-propane. | | | | | |
|---|---|---|---|---|---|
| Toxicity | Doses mgI/kg | Concentration mgI/ml | Administered Volume ml/kg | Dead/Treated Ratio | LD₅₀ C.L. 95% mgI/kg |
| | 900 | 300 | 30.0 | 0/10 | |
| a) | 11600 | 300 | 38.7 | 0/10 | > 15000 |
| | 15000 | 300 | 50.0 | 0/10 | |
| b) | 900 | 300 | 3.0 | 0/10 | >900 |
| a): Intravenous administration (14 days of observation) in mice. b): Intracisternal administration (7 days of observation) in rats. | | | | | |

**Table 2**

| Comparison among some preferred compounds of the invention and IODIXANOL. | | |
|---|---|---|
| Compound | Osmolality 37°C. 300 mgI/ml mosm/kg H₂O | Viscosity 37°C. 300 mgI/ml mPa·s |
| A | 141 | 8.5 |
| B | 131 | 8.6 |
| C | 196 | 8.7 |
| A: 1,3-bis-[3-(L-2-hydroxy-propionyl)amino-5-(1,3-dihydroxy-isopropyl)aminocarbonyl-2,4,6-triiodobenzoyl-amino]-2-hydroxy-propane. B: 1-[3-(L-2-hydroxy-propionyl)amino-5-(1,3-dihydroxyisopropyl)aminocarbonyl-2,4,6-triiodo-benzoyl-amino]-3-[3-hydroxyacetylamino-5-(2,3-dihydroxy- propyl)aminocarbonyl-2,4,6-triiodo-benzoyl-amino]-2-hydroxy-propane. C: 1,3-bis-[N-[3,5-bis-(2,3-dihydroxy-propyl)aminocarbonyl-2,4,6-triiodo-phenyl]-acetylamino]-2-hydroxy-propane [IODIXANOL: EP 108638, Example 3] | | |

## Claims (Claims for the following Contracting State(s): AT, BE, CH, DE, DK, FR, GB, IT, LI, LU, NL, SE)

1. Symmetrical and asymmetrical 1,3-bis-[3-(mono- or poly-hydroxy)acylamino-5-(mono- or poly-hydroxyalkyl)aminocarbonyl-2,4,6-triiodo-benzoyl-amino]-hydroxy- or hydroxyalkyl-propanes of general formula (I) 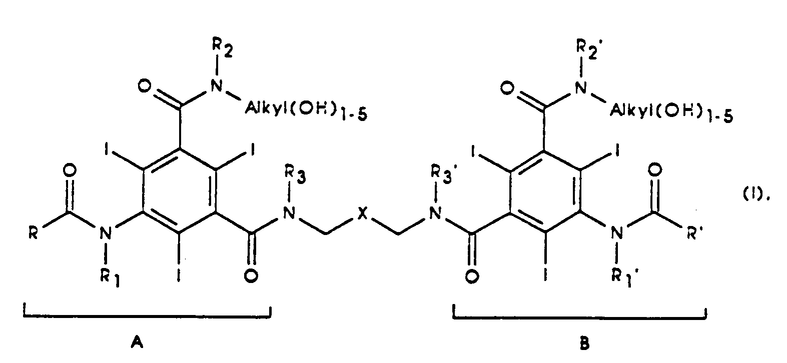 wherein:
R, R', which are the same or different, are a straight or branched mono- or poly-hydroxyalkyl C₁-C₃ residue containing from 1 to 2 OH groups,
R₁, R₂, R₃, which are the same or different, are H or CH₃,
R_{1'}, R_{2'}, R_{3'}, which are the same or different, are H or CH_{3,}
Alkyl(OH)₁₋₅ is one of the groups of formula -CH(CH₂OH)CH(OH)CH₂OH, -CH(CH₂OH)₂, -CH₂CH(OH)-CH₂OH, -CH₂(CHOH)₄CH₂OH, or -CH₂CH₂OH,
X is one of the groups -CH(OH)-, -CH(CH₂OH)-, -C(OH)(CH₂OH)- or -C(CH₂OH)₂-,
A and B, may be the same or different, possible enantiomers, diastereoisomers and/or rotamers thereof.

2. Compounds according to claim 1, where R₂, R₂', R₃, R₃' represent hydrogen and the two residues A and B are equal.

3. Compounds according to claim 1, there R₁, R₁',R₂, R₂', R₃, R₃', represent hydrogen and the two residues A and B are equal.

4. Compounds according to claim 1, where R₂, R₂', R₃, R₃' represent hydrogen, Alkyl(OH)₁₋₅ represents the groups 1,3-dihydroxy-isopropyl or 2,3-dihydroxy-propyl, X represents the group -CH(OH)- and the two residues A and B are equal.

5. A compound according to claims 1-4, selected in the group constituted by:
- 1,3-bis-[3-(2-hydroxy-propionyl)amino-5-(1,3-dihydroxy-isopropyl)aminocarbonyl-2,4,6-triiodo-benzoyl-amino]-2-hydroxy-propane.
- 1,3-bis-[3-(L-2-hydroxy-propionyl)amino-5-(1,3-dihydroxy-isopropyl)aminocarbonyl-2,4,6-triiodo-benzoyl-amino]-2-hydroxy-propane.
- 1,3-bis-[3-hydroxyacetylamino-5-(1,3-dihydroxy-isopropyl)aminocarbonyl-2,4,6-triiodo-benzoyl-amino]-2,2-bis-hydroxymethyl-propane.
- 1,3-bis-[3-(L-2-hydroxy-propionyl)amino-5-(2,3-dihydroxy-propyl)aminocarbonyl-2,4,6-triiodo-benzoyl-amino]-2-hydroxy-propane.
- 1,3-bis-[3-(L-2-hydroxy-propionyl)amino-5-(1,3,4-trihydroxy-2-butyl)aminocarbonyl-2,4,6-triiodo-benzoyl-amino]-2-hydroxy-propane.
- 1-[3-(L-2-hydroxy-propionyl)amino-5-(1,3-dihydroxy-isopropyl)aminocarbonyl-2,4,6-triiodo-benzoyl-amino]-3-[3-hydroxyacetylamino-5-(2,3-dihydroxy-propyl)aminocarbonyl-2,4,6-triiodo-benzoyl-amino]-2-hydroxy-propane.
- 1,3-bis-[3-hydroxyacetylamino-5-(2,3-dihydroxy-propyl)aminocarbonyl-2,4,6-triiodo-benzoyl-amino]- 2,2-bis-hydroxymethyl-propane.
- 1,3-bis-[3-hydroxyacetylamino-5-(1,3,4-trihydroxy-2-butyl)aminocarbonyl-2,4,6-triiodo-benzoyl-amino]-2,2-bis-hydroxymethyl-propane.
- 1,3-bis-[3-(L-2-hydroxy-propionyl)amino-5-[N-methyl-N-(1,3-dihydroxy-isopropyl)]aminocarbonyl-2,4,6-triiodo-benzoyl-amino]-2-hydroxy-propane.
- 1,3-bis-[3-(L-2-hydroxy-propionyl)amino-5-[N-methyl-N-(2,3-dihydroxy-propyl)]aminocarbonyl-2,4,6-triiodo-benzoyl-amino]-2-hydroxy-propane.
- 1,3-bis-[N-methyl-N-[3-(L-2-hydroxy-propionyl)amino-5-(1,3-dihydroxy-isopropyl)aminocarbonyl-2,4,6-triiodo-benzoyl]amino]-2-hydroxy-propane.
- 1,3-bis-[N-methyl-N-[3-(L-2-hydroxy-propionyl)amino-5-(2,3-dihydroxy-propyl)aminocarbonyl-2,4,6-triiodo-benzoyl]amino]-2-hydroxy-propane.
- 1,3-bis-[3-hydroxyacetylamino-5-(1,3-dihydroxy-isopropyl)aminocarbonyl-2,4,6-triiodo-benzoyl-amino]-2-hydroxy-propane.
- 1,3-bis-[3-hydroxyacetylamino-5-(2,3-dihydroxy-propyl)aminocarbonyl-2,4,6-triiodo-benzoyl-amino]-2-hydroxy-propane.
- 1,3-bis-[3-hydroxyacetylamino-5-(1,3,4-trihydroxy-2-butyl)aminocarbonyl-2,4,6-triiodo-benzoyl-amino]-2-hydroxy-propane.
- 1,3-bis-[3-hydroxyacetylamino-5-[N-methyl-N-(1,3-dihydroxy-isopropyl)]aminocarbonyl-2,4,6-triiodo-benzoyl-amino]-2hydroxy-propane.
- 1,3-bis-[3-hydroxyacetylamino-5-[N-methyl-N-(2,3-dihydroxy-propyl)]aminocarbonyl-2,4,6-triiodoben zoyl-amino]-2-hydroxy-propane.
- 1,3-bis-[N-methyl-N-[3-hydroxyacetylamino-5-(1,3-dihydroxy-isopropyl)aminocarbonyl-2,4,6-triiodo-benzoyl]amino]-2-hydroxy-propane.
- 1,3-bis-[N-methy1-N-[3-hydroxyacetylamino-5-(2,3-dihydroxy-propyl)aminocarbonyl-2,4,6-triiodobenzoyl]amino]-2-hydroxy-propane.
- 1,3-bis-[3-(N-methyl-N-hydroxyacetyl)amino-5-(1,3-dihydroxy-isopropyl)aminocarbonyl-2,4,6-triiodo-benzoyl-amino]-2-hydroxy-propane.
- 1,3-bis-[3-[N-methyl-N-(L-2-hydroxy-propionyl)]amino-5-(1,3,4-trihydroxy-2-butyl)aminocarbonyl-2,4,6-triiodo-benzoyl-amino]-2-hydroxy-propane.
- 1,3-bis-[3-[N-methyl-N-(L-2-hydroxy-propionyl)]amino-5-(1,3-dihydroxy-isopropyl)aminocarbonyl-2,4,6-triiodo-benzoyl-amino]-2-hydroxy-propane.
- 1,3-bis-[3-[N-methyl-N-(L-2-hydroxy-propionyl)]amino-5-(2,3-dihydroxy-propyl)aminocarbonyl-2,4,6-triiodo-benzoyl-amino]-2-hydroxy-propane.
- 1,3-bis-[3-(N-methy1-N-hydroxyacetyl)amino-5-(2,3-dihydroxy-propyl)aminocarbonyl-2,4,6-triiodo-benzoyl-amino]-2-hydroxy-propane.
- 1,3-bis-[3-(N-methyl-N-hydroxyacetyl)amino-5-(1,3,4-trihydroxy-2-butyl)aminocarbonyl-2,4,6-triiodo-benzoyl-amino]-2-hydroxy-propane.
- 1,3-bis-[3-(L-2-hydroxy-propionyl)amino-5-(1,3-dihydroxy-isopropyl)aminocarbonyl-2,4,6-triiodo-benzoyl-amino]-2,2-bis-hydroxymethyl-propane.
- 1,3-bis-[3-(L-2-hydroxy-propionyl)amino-5-(2,3-dihydroxy-propyl)aminocarbonyl-2,4,6-triiodo-benzoyl-amino]-2,2-bis-hydroxymethyl-propane.
- 1,3-bis-[3-(L-2-hydroxy-propionyl)amino-5-(1,3,4-trihydroxy-2-butyl)aminocarbonyl-2,4,6-triiodo-benzoyl-amino]-2,2-bis-hydroxymethyl-propane.
- 1,3-bis-[3-[N-methyl-N-(L-2-hydroxy-propionyl)]amino-5-(1,3-dihydroxy-isopropyl)aminocarnonyl-2,4,6-triiodo-benzoyl-amino]-2,2-bis-hydroxymethyl-propane.
- 1,3-bis-[3-[N-methyl-N-(L-2-hydroxy-propionyl)]amino-5-(2,3-dihydroxy-propyl)aminocarbonyl-2,4,6-triiodo-benzoyl-amino]-2,2-bis-hydroxymethyl-propane.
- 1,3-bis-[3-[N-methyl-N-(L-2-hydroxy-propionyl)]amino-5-(1,3,4-trihydroxy-2-butyl)aminocarbonyl-2,4,6-triiodo-benzoyl-amino]-2,2-bis-hydroxymethyl-propane.
- 1,3-bis-[3-(N-methyl-N-hydroxyacetyl)amino-5-(1,3-dihydroxy-isopropyl)aminocarbonyl-2,4,6-triiodo-benzoyl-amino]-2,2-bis-hydroxymethyl-propane.
- 1,3-bis-[3-(N-methyl-N-hydroxyacetyl)amino-5-(2,3-dihydroxy-propyl)aminocarbonyl-2,4,6-triiodo-benzoyl-amino]-2,2-bis-hydroxymethyl-propane.
- 1,3-bis-[3-(N-methyl-N-hydroxyacetyl)amino-5-(1,3,4-trihydroxy-2-butyl)aminocarbonyl-2,4,6-triiodo-benzoyl-amino]-2,2-bis-hydroxymethyl-propane.
- 1,3-bis-[3-(L-2-hydroxy-propionyl)amino-5-[N-methyl-N-(D-1-deoxy-glucitol)]aminocarbonyl-2,4,6-triiodo-benzoyl-amino]-2-hydroxy-propane.
- 1,3-bis-[3-(L-2-hydroxy-propionyl)amino-5-[N-methyl-N-(D-1-deoxy-glucitol)]aminocarbonyl-2,4,6-triiodo-benzoyl-amino]-2,2-bis-hydroxymethyl-propane.
- 1-[3-hydroxyacetylamino-5-(1,3,4-trihydroxy-2-butyl)aminocarbonyl-2,4,6-triiodo-benzoyl-amino]-3-[3-(L-2-hydroxy-propionyl)amino-5-(1,3-dihydroxy-isopropyl)aminocarbonyl-2,4,6-triiodo-benzoyl-amino]-2-hydroxy-propane.
- 1-[3-(L-2-hydroxy-propionyl)amino-5-(1,3-dihydroxy-isopropyl)aminocarbonyl-2,4,6-triiodo-benzoyl-amino]-3-[3-(L-2-hydroxy-propionyl)amino-5-(2,3-dihydroxy-propyl)aminocarbonyl-2,4,6-triiodobenzoyl-amino]-2-hydroxy-propane.
- 1-[3-(L-2-hydroxy-propionyl)amino-5-(1,3-dihydroxy-isopropyl)aminocarbonyl-2,4,6-triiodo-benzoyl-amino]-3-[3-(L-2-hydroxy-propionyl)amino-5-[N-methyl-N-(D-1-deoxy-glucitol)]aminocarbonyl-2,4,6-triiodo-benzoyl-amino]-2-hydroxy-propane.
- 1,3-bis-[N-methyl-N-[3-hydroxyacetylamino-5-(1,3,4-trihydroxy-2-butyl)aminocarbonyl-2,4,6-triiodo-benzoyl]amino]-2-hydroxy-propane.
- 1,3-bis-[N-methyl-N-[3-hydroxyacetylamino-5-[N-methyl-N-(1,3-dihydroxy-isopropyl)]aminocarbonyl-2,4,6-triiodo-benzoyl]amino]-2-hydroxy-propane.
- 1,3-bis-[N-methyl-N-[3-hydroxyacetylamino-5-[N-methyl-N-(D-1-deoxy-glucitol)]aminocarbonyl-2,4,6-triiodo-benzoyl]amino]-2-hydroxy-propane.
- 1,3-bis-[N-methyl-N-[3-(L-2-hydroxy-propionyl)amino-5-(2-hydroxy-ethyl)aminocarbonyl-2,4,6-triiodo-benzoyl]amino]-2-hydroxy-propane.
- 1,3-bis-[N-methyl-N-[3-(L-2-hydroxy-propionyl)amino-5-(1,3,4-trihydroxy-2-butyl)aminocarbonyl-2,4,6-triiodo-benzoyl]amino]-2-hydroxy-propane.
- 1,3-bis-[N-methyl-N-[3-[N-methyl-N-(L-2-hydroxy-propionyl)]amino-5-(2-hydroxy-ethyl)aminocarbonyl-2,4,6-triiodo-benzoyl]amino]-2-hydroxy-propane.
- 1,3-bis-[N-methyl-N-[3-[N-methyl-N-(L-2-hydroxy-propionyl)]amino-5-(1,3-dihydroxy-ispropyl)aminocarbonyl-2,4,6-triiodo-benzoyl]amino]-2-hydroxy-propane.

6. Process for the preparation of the 1,3-bis-[3-(mono- or poly-hydroxy)acylamino-5-(mono- or polyhydroxyalkyl)aminocarbonyl-2,4,6-triiodo-benzoyl-amino]-hydroxy- or hydroxyalkyl-propane of general formula (I) according to claim 1, characterized in that a 1,3-diamino-hydroxy- or hydroxyalkyl-propane of general formula (II)
R₃-HN-CH₂-X-CH₂-NH-R₃' (II)
wherein R₃ and R₃' are H or CH₃, X is one of the groups -CH(OH)-, -CH(CH₂OH)-, -C(OH)(CH₂OH)- or -C(CH₂OH)₂- and one of the amino groups may be protected by a suitable protective group, is reacted, directly or by a multi-step process, with a reactive derivative of a 3-(mono- or poly-acyloxy)acylamino-5-(mono- or poly-hydroxyalkyl)aminocarbonyl-2,4,6-triiodobenzoic acid of general formula (III) 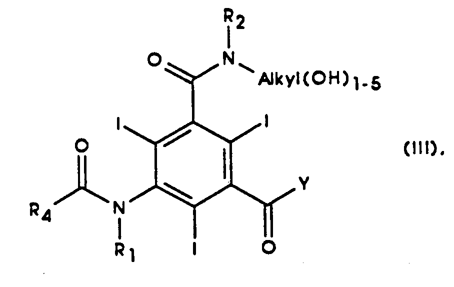 wherein:
R₁, R₂ represent H or CH₃,
R₄ is a straight or branched mono- or poly-acyloxyalkyl C₁-C₁₃ residue containing from 3 to 13 atoms of C and from 1 to 2 lower acyloxy groups C₂-C₅,
Alkyl(OH)₁₋₅ is one of the groups of formula -CH₂(CHOH)₄CH₂OH, -CH₂CH(OH)CH₂OH, -CH(CH₂OH)CH-(OH)CH₂OH, -CH₂CH₂OH, -CH(CH₂OH)₂, or in which the hydroxy groups may be protected preferably by acetalic or ketalic groups,
CO-Y is the residue of a mixed anhydride or, preferably, a halogenocarbonyl group,
to give the product of general formula (IV) 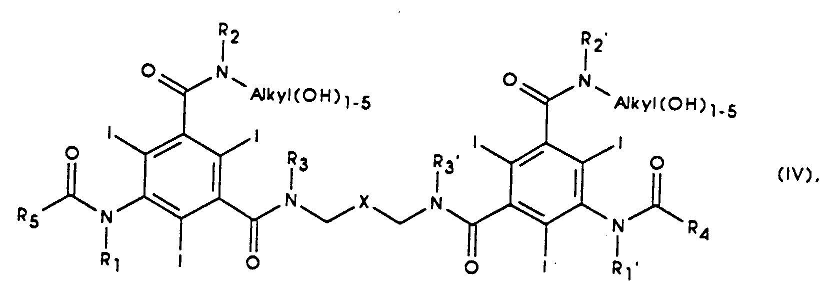 wherein R₁, R₂, R₃, R₁', R₂', R₃', R₄, Alkyl(OH)₁₋₅ and X have the previously described meaning and R₅ may be R or R₄, according to one of the following procedures:
a) a compound of general formula (II) is reacted with a compound of general formula (III) in a molar ratio of 1:2 in a solvent and in the presence of a basic condensation agent to directly obtain a compound of formula (IV) where R₅ corresponds to R₄,
b) a compound of formula (II), in which one of the two amino groups is protected by a suitable protective group, is reacted with a compound of formula (III) in a molar ratio of 1:1 in a solvent and in the presence of a basic condensation agent to obtain, after hydrolysis of the protective groups, the corresponding 1-[3-(mono- or poly-hydroxy)acylamino-5-(mono- or poly-hydroxyalkyl)aminocarbonyl-2,4,6-triiiodo-benzoyl-amino]-3-amino-hydroxyalkyl derivative of formula (V) 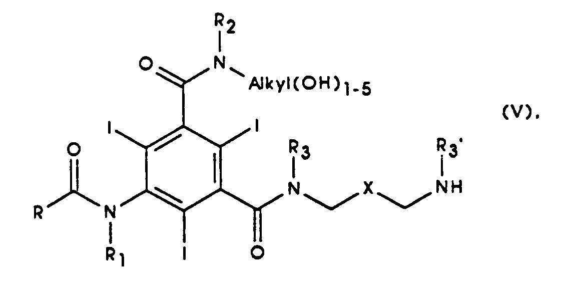
wherein R, R₁, R₂, R₃, R₃', Alkyl(OH)₁₋₅ and X have been previously defined, and this compound (V) is subsequently reacted with a derivative of formula (III) in the presence of a basic condensation agent to obtain the desired compound (IV) where R₅ corresponds to R, then, the compound (IV) obtained through one of the synthetic methods a) or b) is transformed, by hydrolysis of the protective groups, into the corresponding compound of formula (I) and this last one, if the two R₁ and R₁' are H, may be, if desired, N-methylated in an alkaline medium.

7. Process for the preparation of compounds of general formula (I), where R₁ and R₁' are CH₃, characterized in that a compound of general formula (I), in which R₁ and R₁' are hydrogen, is methylated in an alkaline solution by means of treatment with methyl halide, dimethylsulphate, methylsulphonate, dimethylcarbonate.

8. Non-ionic X-ray contrast agents containing as an opacifying component at least one 1,3-bis-[3-(mono- or poly-hydroxy)acylamino-5-(mono- or poly-hydroxyalkyl)aminocarbonyl-2,4,6-triiodo-benzoyl-amino]-hydroxy or hydroxyalkyl-propane according to claims 1 to 5.

## Claims (Claims for the following Contracting State(s): ES, GR)

1. Process for the preparation of symmetrical and asymmetrycal 1,3-bis-[3-(mono- or poly-hydroxy)acylamino-5-(mono- or poly-hydroxyalkyl)aminocarbonyl-2,4,6-triiodo-benzoyl-amino]-hydroxy- or hydroxyalkyl-propanes of general formula (I) 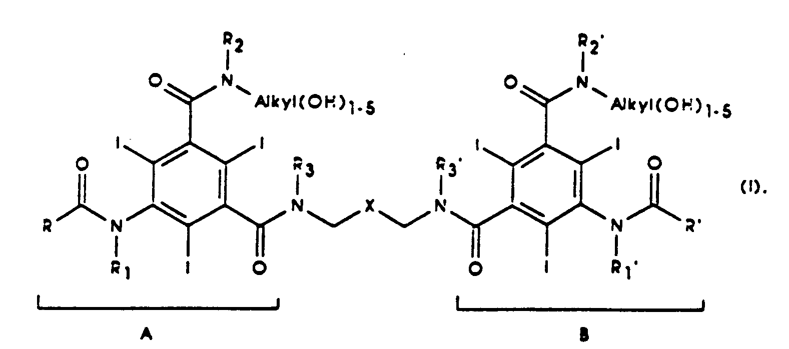 wherein:
R, R', which are the same or different, are a straight or branched mono- or poly-hydroxyalkyl C₁-C₃ residue containing from 1 to 2 OH groups,
R₁, R₂, R₃, which are the same or different, are H or CH₃,
R_{1'}, R_{2'}, R_{3'}, which are the same or different, are H or CH_{3,}
Alkyl(OH)₁₋₅ is one of the groups of formula -CH(CH₂OH)CH(OH)CH₂OH, -CH(CH₂OH)₂, -CH₂CH(OH)-CH₂OH, -CH₂(CHOH)₄CH₂OH, or -CH₂CH₂OH,
X is one of the groups -CH(OH)-, -CH(CH₂OH)-, -C(OH)(CH₂OH)- or -C(CH₂OH)₂-,
A and B, may be the same or different, possible enantiomers, diastereoisomers and/or rotamers thereof.
characterized in that a 1,3-diamino-hydroxy- or hydroxyalkyl-propane of general formula (II)
R₃-HN-CH₂-X-CH₂-NH-R₃' (II)
wherein R₃ and R₃' are H or CH₃, X is one of the groups -CH(OH)-, -CH(CH₂OH)-, -C(OH)(CH₂OH)- or -C(CH₂OH)₂- and one of the amino groups may be protected by a suitable protective group, is reacted, directly or by a multi-step process, with a reactive derivative of a 3-(mono- or poly-acyloxy)acylamino-5-(mono- or poly-hydroxyalkyl)aminocarbonyl-2,4,6-triiodobenzoic acid of general formula (III) 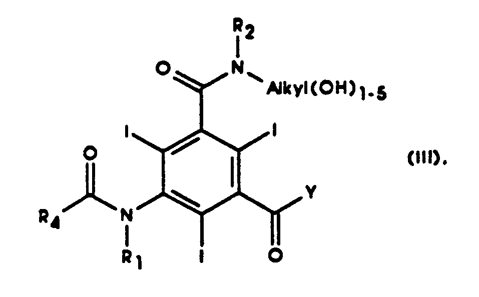 wherein:
R₁, R₂ represent H or CH₃,
R₄ is a straight or branched mono- or poly-acyloxyalkyl C₁-C₁₃ residue containing from 3 to 13 atoms of C and from 1 to 2 lower acyloxy groups C₂-C₅,
Alkyl(OH)₁₋₅ is one of the groups of formula -CH₂(CHOH)₄CH₂OH, -CH₂CH(OH)CH₂OH, -CH(CH₂OH)CH-(OH)CH₂OH, -CH₂CH₂OH, -CH(CH₂OH)₂, or in which the hydroxy groups may be protected preferably by acetalic or ketalic groups,
CO-Y is the residue of a mixed anhydride or, preferably, a halogenocarbonyl group,
to give the product of general formula (IV) 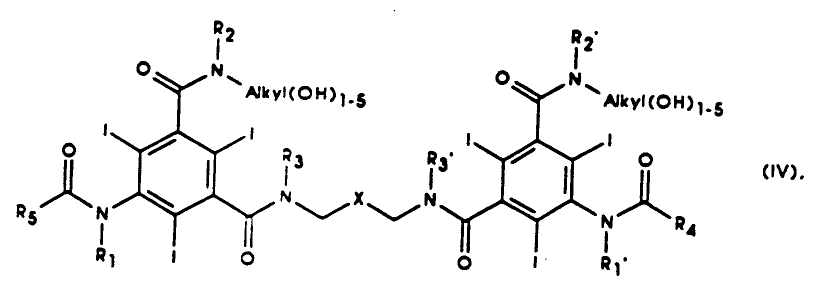 wherein R₁, R₂, R₃, R₁', R₂', R₃', R₄, Alkyl(OH)₁₋₅ and X have the previously described meaning and R₅ may be R or R₄, according to one of the following procedures:
a) a compound of general formula (II) is reacted with a compound of general formula (III) in a molar ratio of 1:2 in a solvent and in the presence of a basic condensation agent to directly obtain a compound of formula (IV) where R₅ corresponds to R₄,
b) a compound of formula (II), in which one of the two amino groups is protected by a suitable protective group, is reacted with a compound of formula (III) in a molar ratio of 1:1 in a solvent and in the presence of a basic condensation agent to obtain, after hydrolysis of the protective groups, the corresponding 1-[3-(mono- or poly-hydroxy)acylamino-5-(mono- or poly-hydroxyalkyl)aminocarbonyl-2,4,6-triiiodo-benzoyl-amino]-3-amino-hydroxyalkyl derivative of formula (V) 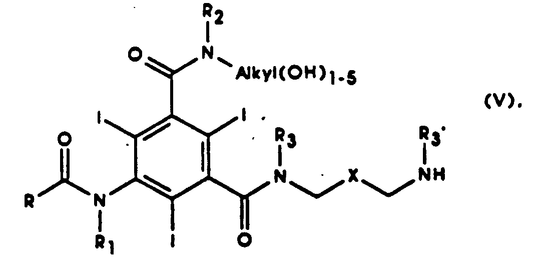
wherein R, R₁, R₂, R₃, R₃', Alkyl(OH)₁₋₅ and X have been previously defined, and this compound (V) is subsequently reacted with a derivative of formula (III) in the presence of a basic condensation agent to obtain the desired compound (IV) where R₅ corresponds to R, then, the compound (IV) obtained through one of the synthetic methods a) or b) is transformed, by hydrolysis of the protective groups, into the corresponding compound of formula (I) and this last one, if the two R₁ and R₁' are H, may be, if desired, N-methylated in an alkaline medium.

2. Process for the preparation of compounds of general formula (I), where R₁ and R₁' are CH₃, characterized in that a compound of general formula (I), in which R₁ and R₁' are hydrogen, is methylated in an alkaline solution by means of treatment with methyl halide, dimethylsulphate, methylsulphonate, dimethylcarbonate.

## Patentansprüche (Patentansprüche für folgende(n) Vertragsstaat(en): AT, BE, CH, DE, DK, FR, GB, IT, LI, LU, NL, SE)

1. Symmetrische und asymmetrische 1,3-Bis-[3-(mono-oder polyhydroxy)acylamino-5-(mono- oder polyhydroxyalkyl)-aminocarbonyl-2,4,6-triiodobenzoylamino]-hydroxy-oder-hydroxyalkylpropane der allgemeinen Formel (I) 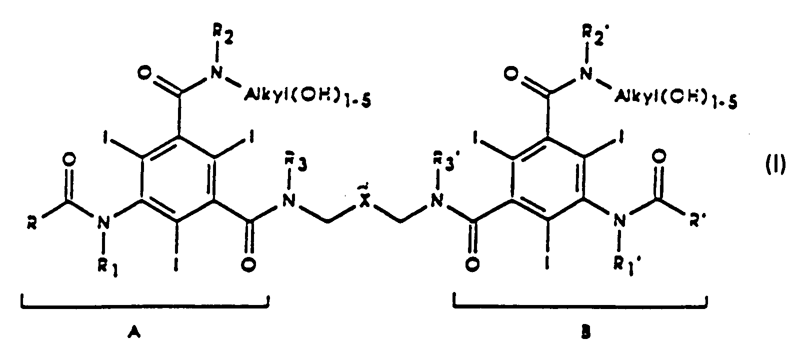 wobei die Reste R und R' gleich oder voneinander verschieden sein können und unverzweigte oder verzweigte Mono- oder Polyhydroxyalkylreste mit 1 bis 3 C-Atomen und 1 bis 2 Hydroxylgruppen sind,
die Reste R₁, R₂, R₃, R'₁, R'₂ und R'₃, die gleich oder voneinander verschieden sein können, Wasserstoffatome oder CH₃-Gruppen sind,
der Rest Alkyl(OH)₁₋₅ ausgewählt ist aus -CH(CH₂OH)CH(OH)CH₂OH,
-CH(CH₂OH)₂, -CH₂CH(OH)CH₂OH, -CH₂(CHOH)₄CH₂OH oder
-CH₂CH₂OH,
der Rest X ausgewählt ist aus -CH(OH)-, -CH(CH₂OH)-, -C(OH)(CH₂OH)- oder -C(CH₂OH)₂-,
die Reste A und B gleich oder voneinander verschieden sind und Enantiomere, Diastereomere und/oder Rotamere sein können.

2. Verbindungen nach Anspruch 1, wobei die Reste R₂, R'₂, R₃ und R'₃ Wasserstoffatome sind und die beiden Reste A und B gleich sind.

3. Verbindungen nach Anspruch 1, wobei die Reste R₁, R'₁, R₂, R'₂, R₃ und R'₃ Wasserstoffatome sind und die beiden Reste A und B gleich sind.

4. Verbindungen nach Anspruch 1, wobei die Reste R₂, R'₂, R₃ und R'₃ Wasserstoffatome sind, der Rest Alkyl(OH)₁₋₅ ein 1,3-Dihydroxyisopropylrest oder 2,3-Dihydroxypropylrest ist, der Rest X -CH(OH)- ist und die beiden Reste A und B gleich sind.

5. Verbindung nach einem der Ansprüche 1 bis 4, ausgewählt aus
1,3-Bis-[3-(2-hydroxypropionyl)-amino-5-(1,3-dihydroxyisopropyl)-aminocarbonyl-2,4,6-triiodobenzoylamino)-2-hydroxypropan,
1,3-Bis-[3-(L-2-hydroxypropionyl)-amino-5-(1,3-dihydroxyisopropyl)-aminocarbonyl-2,4,6-triiodobenzoylamino]-2-hydroxypropan,
1,3-Bis-[3-hydroxyacetylamino-5-(1 ,3-dihydroxyisopropyl)-aminocarbonyl-2,4,6-triiodobenzoylamino]-2,2-bis-hydroxymethylpropan,
1,3-Bis-[3-(L-2-hydroxypropionyl)-amino-5-(2,3-dihydroxypropyl)-aminocarbonyl-2,4,6-triiodobenzoylamino]-2-hydroxypropan,
1,3-Bis-[3-(L-2-hydroxypropionyl)-amino-5-(1,3,4-trihydroxy-2-butyl)-aminocarbonyl-2,4,6-triiodobenzoylamino]-2-hydroxypropan,
1-[3-(L-2-hydroxypropionyl)-amino-5-(1,3-dihydroxyisopropyl)-aminocarbonyl-2,4,6-triiodobenzoylamino]-3-[3-hydroxyacetylamino-5-
(2,3-dihydroxypropyl)-aminocarbonyl-2,4,6-triiodo-benzoylamino]-2-hydroxypropan,
1,3-Bis-[3-hydroxyacetylamino-5-(2,3-dihydroxypropyl)-aminocarbonyl-2,4,6-triiodobenzoylamino]-2,2-bis-hydroxymethylpropan,
1,3-Bis-[3-hydroxyacetylamino-5-(1,3,4-trihydroxy-2-butyl)-aminocarbonyl-2,4,6-triodobenzoylamino]-2,2-bis-hydroxymethylpropan,
1,3-Bis-[3-(L-2-hydroxypropionyl)-amino-5-(N-methyl-N-(1,3-dihydroxyisopropyl)]-aminocarbonyl-2,4,6-triiodobenzoylamino]-2-hydroxypropan,
1,3-Bis-[3-(L-2-hydroxypropionyl)-amino-5-[N-methyl-N-(2,3-dihydroxypropyl)]-aminocarbonyl-2,4,6-triiodobenzoylamino]-2-hydroxypropan,
1,3-Bis-[N-methyl-N-[3-(L-2-hydroxypropionyl)-amino-5-(1,3-dihydroxyisopropyl)-aminocarbonyl-2,4,6-triiodobenzoylamino]-2-hydroxypropan,
1,3-Bis-[N-methyl-N-[3-(L-2-hydroxypropionyl)-amino-5-[2,3-dihydroxypropyl)-aminocarbonyl-2,4,6-triiodobenzoylamino]-2-hydroxypropan,
1,3-Bis-[3-hydroxyacetylamino-5-(1,3-dihydroxyisopropyl)-aminocarbonyl-2,4,6-triiodobenzoylamino]-2-hydroxypropan,
1,3-Bis-[3-hydroxyacetylamino-5-(2,3-dihydroxypropyl)-aminocarbonyl-2,4,6-triiodobenzoylamino]-2-hydroxypropan,
1,3-Bis-[3-hydroxyacetylamino-5-(1,3,4-trihydroxy-2-butyl)-aminocarbonyl-2,4,6-triiodobenzoylamino]-2-hydroxypropan,
1,3-Bis-[3-hydroxyacetylamino-5-[N-methyl-N-(1,3-dihydroxyisopropyl)]-aminocarbonyl-2,4,6-triiodobenzoylamino]-2-hydroxypropan,
1,3-Bis-[3-hydroxyacetylamino-5-[N-methyl-N-(2,3-dihydroxypropyl)]-aminocarbonyl-2,4,6-triiodobenzoylamino]-2-hydroxypropan,
1,3-Bis-[N-methyl-N-[3-hydroxyacetylamino-5-(1,3-dihydroxyisopropyl)-aminocarbonyl-2,4,6-triiodobenzoylamino]-2-hydroxypropan,
1,3-Bis-[N-methyl-N-[3-hydroxyacetylamino-5-(2,3-dihydroxypropyl)-aminocarbonyl-2,4,6-triiodobenzoylamino]-2-hydroxypropan,
1,3-Bis-[3-(N-methyl-N-hydroxyacetyl)-amino-5-(1,3-dihydroxyisopropyl)-aminocarbonyl-2,4,6-triiodobenzoylamino]-2-hydroxypropan,
1,3-Bis-[3-[N-methyl-N-(L-2-hydroxypropionyl)]-amino-5-(1,3,4-trihydroxy-2-butyl)-aminocarbonyl-2,4,6-triiodobenzoylamino]-2-hydroxypropan,
1,3-Bis-[3[N-methyl-N-(L-2-hydroxypropionyl)]-amino-5-(1,3-dihydroxyisopropyl)-aminocarbonyl-2,4,6-triiodobenzoylamino]-2-hydroxypropan,
1,3-Bis-[3-[N-methyl-N(L-2-hydroxypropionyl)]-amino-5-(2,3-dihydroxypropyl)-aminocarbonyl-2,4,6-triiodobenzoylamino]-2-hydroxypropan,
1,3-Bis-[3-(N-methyl-N-hydroxyacetyl)-amino-5-(2,3-dihydroxypropyl)-aminocarbonyl-2,4-6-triiodobenzoylamino]-2-hydroxypropan,
1,3-Bis-[3-(N-methyl-N-hydroxyacetyl)-amino-5-(1,3,4-trihydroxy-2-butyl)-aminocarbonyl-2,4,6-triiodobenzoylamino]-2-hydroxypropan,
1,3-Bis-[3-(L-2-hydroxypropionyl)-amino-5-(1,3-dihydroxyisopropyl)-aminocarbonyl-2,4,6-triiodobenzoylamino]-2,2-bis-hydroxymethylpropan,
1,3-Bis-[3-(L-2-hydroxypropionyl)-amino-5-(2,3-dihydroxypropyl)-aminocarbonyl-2,4,6-triiodobenzoylamino]-2,2-bis-hydroxymethylpropan,
1,3-Bis-[3-(L-2-hydroxypropionyl)-amino-5-(1,3,4-trihydroxy-2-butyl)-aminocarbonyl-2,4,6-triiodobenzoylamino]-2,2-bis-hydroxymethylpropan,
1,3-Bis[3-[N-methyl-N-(L-2-hydroxypropionyl)]-amino-5-(1,3-dihydroxyisopropyl)-aminocarbonyl-2,4,6-triiodobenzoylamino]-2,2-bis-hydroxymethylpropan,
1,3-Bis-[3-[N-methyl-N-(L-2-hydroxypropionyl)]-amino-5-(2,3-dihydroxypropyl)-aminocarbonyl-2,4,6-triiodobenzoylamino]-2,2-bis-hydroxymethylpropan,
1,3-Bis-[3-[N-methyl-N-(L-2-hydroxypropionyl)]-amino-5-(1,3,4-trihydroxy-2-butyl)-aminocarbonyl-2,4,6-triiodobenzoylamino]-2,2-bis-hydroxymethylpropan,
1,3-Bis-[3-(N-methyl-N-hydroxyacetyl)-amino-5-(1,3-dihydroxyisopropyl)-aminocarbonyl-2,4,6-triiodobenzoylamino]-2,2-bis-hydroxymethylpropan,
1,3-Bis-[3-(N-methyl-N-hydroxyacetyl)-amino-5-(2,3-dihydroxypropyl)-aminocarbonyl-2,4,6-triiodobenzoylamino]-2,2-bis-hydroxymethylpropan,
1,3-Bis-[3-(N-methyl-N-hydroxyacetyl)-amino-5-(1,3,4-trihydroxy-2-butyl)-aminocarbonyl-2,4,6-triiodobenzoylamino]-2,2-bis-hydroxymethylpropan,
1,3-Bis-[3-(L-2-hydroxypropionyl)-amino-5-[N-methyl-N-(D-1-deoxyglucit)]-aminocarbonyl-2,4,6-triiodobenzoylamino]-2-hydroxypropan,
1,3-Bis-[3-(L-2-hydroxypropionyl)-amino-5-[N-methyl-N-(D-1-deoxyglucit)]-aminocarbonyl-2,4,6-triiodobenzoylamino]-2,2-bis-hydroxymethylpropan,
1-(3-hydroxyacetylamino-5-(1,3,4-trihydroxy-2-butyl)-aminocarbonyl-2,4,6-triiodobenzoylamino]-3-[3-(L-2-hydroxypropionyl)-amino-5-(1,3-dihydroxyisopropyl)-aminocarbonyl-2,4,6-triiodobenzoylamino]-2-hydroxypropan,
1-[3-(L-2-hydroxypropionyl)-amino-5-(1,3dihydroxyisopropyl))-aminocarbonyl-2,4,6-triiodobenzoylamino]-3-[3-(L-2-hydroxypropionyl)-amino-5-(2,3-dihydroxypropyl)-aminocarbonyl-2,4,6-triiodobenzoylamino]-2-hydroxypropan,
1-[3-(L-2-hydroxypropionyl)-amino-5-(1,3-dihydroxyisopropyl))-aminocarbonyl-2,4,6-triiodobenzoylamino]-3-[3-(L-2-hydroxypropionyl)-amino-5-[N-methyl-N-(D-1-deoxyglucit)-aminocarbonyl-2,4,6-triiodobenzoylamino]-2-hydroxypropan,
1,3-Bis-[N-methyl-N-[3-hydroxyacetylamino-5-(1,3,4-trihydroxy-2-butyl)]-aminocarbonyl-2,4,6-triiodobenzoylamino]-2-hydroxypropan,
1,3-Bis-[N-methyl-N-[3-hydroxyacetylamino-5-[N-methyl-N-(1,3-dihydroxyisopropyl)]-aminocarbonyl-2,4,6-triiodobenzoylamino]-2-hydroxypropan,
1,3-Bis-[N-methyl-N-[3-hydroxyacetylamino-5-[N-methyl-N-(D-1-deoxyglucit)]-aminocarbonyl-2,4,6-triiodobenzoylamino]-2-hydroxypropan, 1,3-Bis-[N-methyl-N-[3-(L-2-hydroxypropionyl)-amino-5-(2-hydroxyethyl)-aminocarbonyl-2,4,6-triiodobenzoylamino]-2-hydroxypropan,
1,3-Bis-[N-methyl-N-[3-(L-2-hydroxypropionyl)-amino-5-(1,3,4-trihydroxy-2-butyl)-aminocarbonyl-2,4,6-triiodobenzoylamino]-2-hydroxypropan,
1,3-Bis-[N-methyl-N-[3-[N-methyl-N-(L-2-hydroxypropionyl)]-amino-5-(2-hydroxyethyl)-aminocarbonyl-2,4,6-triiodobenzoylamino]-2-hydroxypropan,
1,3-Bis-[N-methyl-N-[3-[N-methyl-N-(L-2-hydroxypropionyl)]-amino-5-(1,3-dihydroxyisopropyl)-aminocarbonyl-2,4,6-triiodobenzoylamino]-2-hydroxypropan.

6. Verfahren zur Herstellung des 1,3-Bis-[3-(mono- oder polyhydroxy)-acylamino-5-(mono- oder polyhydroxyalkyl)-aminocarbonyl-2,4,6-triiodobenzoylamino]-hydroxy- oder hydroxyalkylpropan der allgemeinen Formel (I) nach Anspruch 1, dadurch gekennzeichnet, daß ein 1,3-Diaminohydroxy- oder
-hydroxyalkylpropan der allgemeinen Formel (II)
R₃-HN-CH₂-X-CH₂-NH-R'₃ (II)
wobei die Reste R₃ und R'₃ Wasserstoffatome oder CH₃-Gruppen sind, der Rest X ausgewählt ist aus -CH(OH)-, -CH(CH₂OH)-, -C(OH)(CH₂OH)- oder -C(CH₂OH)₂-, und eine der Aminogruppen durch eine geeignete Schutzgruppe geschützt sein kann,
direkt oder in einem mehrstufigen Verfahren mit einem reaktiven Derivat einer 3-(Mono- oder Polyacyloxy)-acylamino-5-(mono- oder polyhydroxyalkyl)-aminocarbonyl-2,4,6-triiodobenzoesäure der allgemeinen Formel (III) 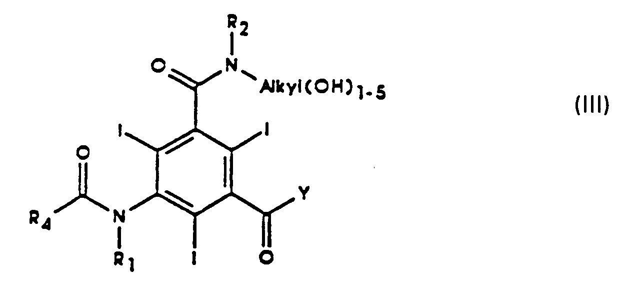 umgesetzt wird, wobei
die Reste R₁ und R₂ Wasserstoffatome oder CH₃-Gruppen sind, der Rest R₄ ein unverzweigter oder verzweigter Mono- oder Polyacyloxyalkylrest mit 1 bis 13 C-Atomen und 1 bis 2 niederen-C₂ bis C₅ Acyloxyresten ist, der Rest Alkyl(OH)₁₋₅ ausgewählt ist aus -CH₂(CHOH)₄CH₂OH,
-CH₂CH(OH)CH₂OH, -CH(CH₂OH)CH(OH)CH₂OH, -CH₂CH₂OH,
-CH(CH₂OH)₂, wobei wahlweise die Hydroxylgruppen geschützt sein können, vorzugsweise durch Acetal- oder Ketalreste, der Rest CO-Y ein gemischter Anhydridrest oder, vorzugsweise, ein Halogencarbonylrest ist, um ein Produkt der allgemeinen Formel (IV) zu erhalten 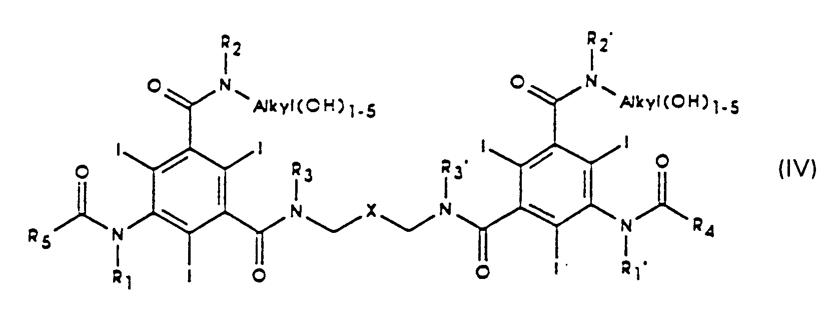 wobei die Reste R₁, R₂, R₃, R'₁, R'₂, R'₃, R₄, Alkyl(OH)₁₋₅ und X die vorstehend angegebene Bedeutung haben und der Rest R₅ gleich dem Rest R oder R₄ sein kann, entweder
a) durch Umsetzung einer Verbindung der allgemeinen Formel (II) mit einer Verbindung der allgemeinen Formel (III) in einem Molverhältnis von 1:2 in einem Lösungsmittel in Gegenwart eines basischen Kondensationsmittels, um direkt eine Verbindung der allgemeinen Formel (IV) zu erhalten, worin der Rest R₅ gleich dem Rest R₄ ist, oder
b) durch Umsetzung einer Verbindung der allgemeinen Formel (II), in der eine der beiden Aminogruppen mit einer geeigneten Schutzgruppe geschützt ist, mit einer Verbindung der allgemeinen Formel (III) im Molverhältnis von 1:1 in einem Lösungsmittel in Gegenwart eines basischen Kondensationsmittels, um nach der Hydrolyse der Schutzgruppen das entsprechende 1-[3-(Mono- oder Polyhydroxy)-acylamino-5-(mono- oder polyhydroxyalkyl)-aminocarbonyl-2,4,6-triiodobenzoylamino]-3-aminohydroxyalkyl-Derivat der allgemeinen Formel (V) zu erhalten 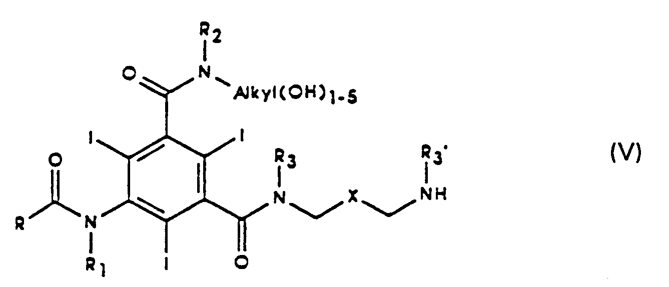 wobei die Reste R, R₁, R₂, R₃, R'₃, Alkyl(OH)₁₋₅ und X die vorstehend angegebene Bedeutung haben und die Verbindung der allgemeinen Formel (V) anschließend mit einem Derivat der allgemeinen Formel (III) in Gegenwart eines basischen Kondensationsmittels umgesetzt wird, um die gewünschte Verbindung (IV) zu erhalten, worin der Rest R₅ gleich dem Rest R ist,
und anschließende Überführung der nach einem der Syntheseverfahren a) oder b) erhaltenen Verbindung der allgemeinen Formel (IV) durch Hydrolyse der Schutzgruppen in die entsprechende Verbindung der allgemeinen Formel (I) und gegebenenfalls N-Methylierung dieser Verbindung der allgemeinen Formel (I) in einem alkalischen Medium, sofern die beiden Reste R₁ und R'₁ Wasserstoffatome sind.

7. Verfahren zur Herstellung von Verbindungen der allgemeinen Formel (I), wobei die Reste R₁ und R'₁ CH₃-Gruppen sind, dadurch gekennzeichnet, daß eine Verbindung der allgemeinen Formel (I), in der die Reste R₁ und R'₁ Wasserstoffatome sind, durch Umsetzung mit einem Methylhalogenid, Dimethylsulfat, Methylsulfonat oder Dimethylcarbonat in einer alkalischen Lösung methyliert wird.

8. Nicht-ionische Röntgenkontrastmittel, die als Trübungskomponente (Kontrastkomponente) mindestens ein 1,3-Bis-[3-(mono- oder polyhydroxy)-acylamino-5-(mono-oder polyhydroxyalkyl)-aminocarbonyl-2,4,6-triiodobenzoylamino]-hydroxy oder-hydroxyalkylpropan nach einem der Ansprüche 1 bis 5 enthalten.

## Patentansprüche (Patentansprüche für folgende(n) Vertragsstaat(en): ES, GR)

1. Verfahren zur Herstellung von symmetrischen und asymmetrischen 1,3-Bis-[3-(mono- oder polyhydroxy)-acylamino-5-(mono- oder polyhydroxyalkyl)-aminocarbonyl-2,4,6-triiodobenzoylamino]-hydroxy- oder -hydroxyalkylpropanen der allgemeinen Formel (I) 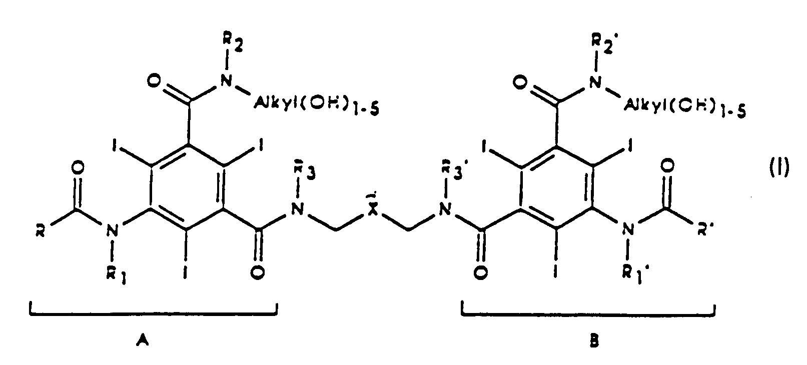 wobei die Reste R und R' gleich oder voneinander verschieden sein können und unverzweigte oder verzweigte Mono- oder Polyhydroxyalkylreste mit 1 bis 3 C-Atomen und 1 bis 2 Hydroxylgruppen sind,
die Reste R₁, R₂, R₃, R'₁, R'₂ und R'₃, die gleich oder voneinander verschieden sein können, Wasserstoffatome oder CH₃-Gruppen sind,
der Rest Alkyl(OH)₁₋₅ ausgewählt ist aus -CH(CH₂OH)CH(OH)CH₂OH,
-CH(CH₂OH)₂, -CH₂CH(OH)CH₂OH, -CH₂(CHOH)₄CH₂OH oder
-CH₂CH₂OH,
der Rest X ausgewählt ist aus -CH(OH)-, -CH(CH₂OH)-, -C(OH)(CH₂OH)- oder -C(CH₂OH)₂-,
die Reste A und B gleich oder voneinander verschieden sind und Enantiomere, Diastereomere und/oder Rotamere sein können, dadurch gekennzeichnet, daß ein 1,3-Diaminohydroxy- oder
-hydroxyalkylpropan der allgemeinen Formel (II)
R₃-HN-CH₂-X-CH₂-NH-R'₃ (II)
wobei die Reste R₃ und R'₃ Wasserstoffatome oder CH₃-Gruppen sind, der Rest X ausgewählt ist aus -CH(OH)-, -CH(CH₂OH)-, -C(OH)(CH₂OH)- oder -C(CH₂OH)₂-, und eine der Aminogruppen durch eine geeignete Schutzgruppe geschützt sein kann,
direkt oder in einem mehrstufigen Verfahren mit einem reaktiven Derivat einer 3-(Mono- oder Polyacyloxy)-acylamino-5-(mono- oder polyhydroxyalkyl)-aminocarbonyl-2,4,6-triiodobenzoesäure der allgemeinen Formel (III) 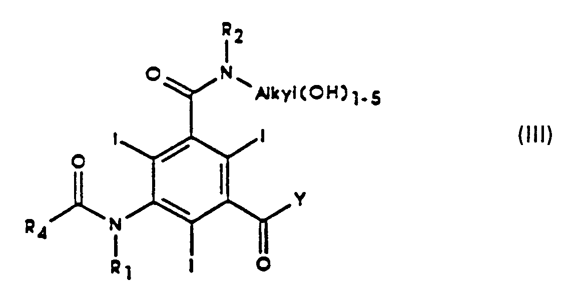 umgesetzt wird, wobei
die Reste R₁ und R₂ Wasserstoffatome oder CH₃-Gruppen sind, der Rest R₄ ein unverzweigter oder verzweigter Mono- oder Polyacyloxyalkylrest mit 1 bis 13 C-Atomen und 1 bis 2 niederen-C₂ bis C₅ Acyloxyresten ist, der Rest Alkyl(OH)₁₋₅ ausgewählt ist aus -CH₂(CHOH)₄CH₂OH,
-CH₂CH(OH)CH₂OH, -CH(CH₂OH)CH(OH)CH₂OH, -CH₂CH₂OH,
-CH(CH₂OH)₂, wobei wahlweise die Hydroxylgruppen geschützt sein können, vorzugsweise durch Acetal- oder Ketalreste, der Rest CO-Y ein gemischter Anhydridrest oder, vorzugsweise, ein Halogencarbonylrest ist, um ein Produkt der allgemeinen Formel (IV) zu erhalten 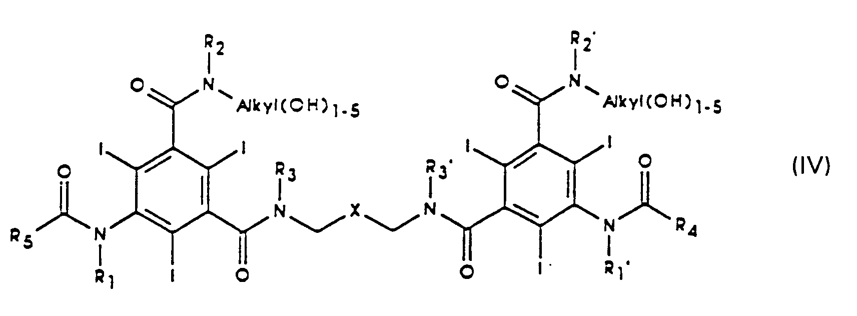 wobei die Reste R₁, R₂, R₃, R'₁, R'₂, R'₃, R₄, Alkyl(OH)₁₋₅ und X die vorstehend angegebene Bedeutung haben und der Rest R₅ gleich dem Rest R oder R₄ sein kann, entweder
a) durch Umsetzung einer Verbindung der allgemeinen Formel (II) mit einer Verbindung der allgemeinen Formel (III) in einem Molverhältnis von 1:2 in einem Lösungsmittel in Gegenwart eines basischen Kondensationsmittels, um direkt eine Verbindung der allgemeinen Formel (IV) zu erhalten, worin der Rest R₅ gleich dem Rest R₄ ist, oder
b) durch Umsetzung einer Verbindung der allgemeinen Formel (II), in der eine der beiden Aminogruppen mit einer geeigneten Schutzgruppe geschützt ist, mit einer Verbindung der allgemeinen Formel (III) im Molverhältnis von 1:1 in einem Lösungsmittel in Gegenwart eines basischen Kondensationsmittels, um nach der Hydrolyse der Schutzgruppen das entsprechende 1-[3-(Mono- oder Polyhydroxy)-acylamino-5-(mono- oder polyhydroxyalkyl)-aminocarbonyl-2,4,6-triiodobenzoylamino]-3-aminohydroxyalkyl-Derivat der allgemeinen Formel (V) zu erhalten 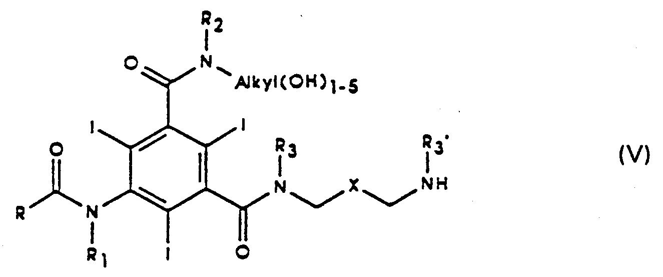 wobei die Reste R, R₁, R₂, R₃, R'₃, Alkyl(OH)₁₋₅ und X die vorstehend angegebene Bedeutung haben und die Verbindung der allgemeinen Formel (V) anschließend mit einem Derivat der allgemeinen Formel (III) in Gegenwart eines basischen Kondensationsmittels umgesetzt wird, um die gewünschte Verbindung (IV) zu erhalten, worin der Rest R₅ gleich dem Rest R ist,
und anschließende Überführung der nach einem der Syntheseverfahren a) oder b) erhaltenen Verbindung der allgemeinen Formel (IV) durch Hydrolyse der Schutzgruppen in die entsprechende Verbindung der allgemeinen Formel (I) und gegebenenfalls N-Methylierung dieser Verbindung der allgemeinen Formel (I) in einem alkalischen Medium, sofern die beiden Reste R₁ und R'₁ Wasserstoffatome sind.

2. Verfahren zur Herstellung von Verbindungen der allgemeinen Formel (I), wobei die Reste R₁ und R'₁ CH₃-Gruppen sind, dadurch gekennzeichnet, daß eine Verbindung der allgemeinen Formel (I), in der die Reste R₁ und R'₁ Wasserstoffatome sind, durch Umsetzung mit einem Methylhalogenid, Dimethylsulfat, Methylsulfonat oder Dimethylcarbonat in einer alkalischen Lösung methyliert wird.

## Revendications (Revendications pour l'(les) Etat(s) contractant(s) suivant(s): AT, BE, CH, DE, DK, FR, GB, IT, LI, LU, NL, SE)

1. 1,3-bis[3-(mono- ou poly-hydroxy)acylamino-5-(mono- ou poly-hydroxyalkyl)aminocarbonyl-2,4,6-triiodobenzoylamino]-hydroxy ou hydroxyalkyl-propanes symétriques et asymétriques de formule générale (I) 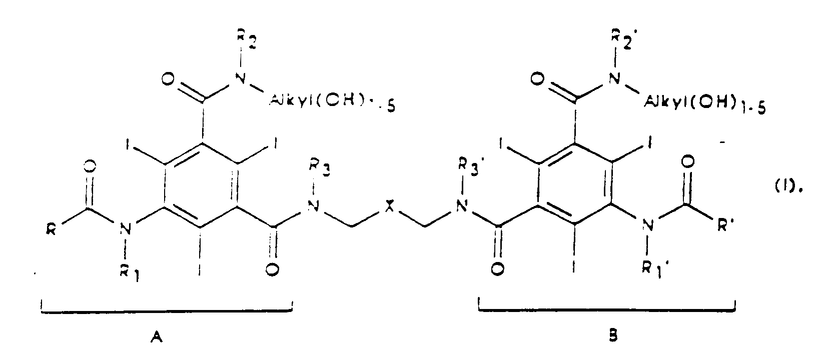 où :
R, R', qui sont identiques ou différents, représentent un résidu mono- ou polyhydroxyalkyle en C₁-C₃ à chaîne droite ou ramifiée contenant 1 ou 2 groupes OH,
R₁, R₂, R₃, qui sont identiques ou différents, sont H ou CH₃,
R₁', R₂', R₃', qui sont identiques ou différents, sont H ou CH₃,
Alkyl(OH)₁₋₅ est l'un des groupes de formule -CH(CH₂OH)CH(OH)CH₂OH, -CH(CH₂OH)₂, -CH₂CH(OH)CH₂OH, -CH₂(CHOH)₄CH₂OH ou -CH₂CH₂OH,
X est l'un des groupes -CH(OH)-, -CH(CH₂OH)-, -C(OH)(CH₂OH)- ou -C(CH₂OH)₂-
A et B, peuvent être identiques ou différents, leurs énantiomères, diastéréoisomères et/ou rotamères possibles.

2. Composés selon la revendication 1, dans lesquels R₂, R₂', R₃, R₃' représentent de l'hydrogène et les deux résidus A et B sont identiques.

3. Composés selon la revendication 1, dans lesquels R₁, R₁', R₂, R₂', R₃, R₃', représentent de l'hydrogène et les deux résidus A et B sont identiques.

4. Composés selon la revendication 1, dans lesquels R₂, R₂', R₃, R₃' représentent de l'hydrogène, Alkyl(OH)₁₋₅ représente les groupes 1,3-dihydroxyisopropyle ou 2,3-dihydroxvpropyle, X représente le groupe -CH(OH)- et les deux résidus A et B sont identiques.

5. Un composé selon les revendications 1 à 4, choisi dans le groupe constitué par les composés suivants :
- 1,3-bis-[3-(2-hydroxy-propionyl)amino-5-(1,3-dihydroxy-isopropyl)aminocarbonyl-2,4,6-triiodo-benzoyl-amino]-2-hydroxy-propane.
- 1,3-bis-[3-(L-2-hydroxy-propionyl)amino-5-(1,3-dihydroxy-isopropyl)aminocarbonyl-2,4,6-triiodo-benzoyl-amino]-2-hydroxy-propane.
- 1,3-bis-[3-hydroxyacétylamino-5-(1,3-dihydroxy-isopropyl)aminocarbonyl-2,4,6-triiodo-benzoyl-amino]-2 2-bis-hydroxyméthyl-propane.
- 1,3-bis-[3-(L-2-hydroxy-propionyl)amino-5-(2,3-dihydroxy-propyl)aminocarbonyl-2,4,6-triiodo-benzoyl-amino]-2-hydroxy-propane.
- 1,3-bis-[3-(L-2-hydroxy-propionyl)amino-5-(1,3,4-trihydroxy-2-butyl)aminocarbonyl-2,4,6-triiodo-benzoyl-amino]-2-hydroxy-propane.
- 1-[3-(L-2-hydroxy-propionyl)amino-5-(1,3-dihydroxy-isopropyl)aminocarbonyl-2,4,6-triiodo-benzoyl-amino]-3-[3-hydroxyacétylamino-5-(2,3-dihydroxy-propyl)aminocarbonyl-2,4,6-triiodo-benzoyl-amino]-2-hydroxy-propane.
- 1,3-bis-[3-hydroxyacétylamino-5-(2,3-dihydroxy-propyl)aminocarbonyl-2,4,6-triiodo-benzoyl-amino]-2,2-bis-hydroxymethyl-propane.
- 1,3-bis-[3-hydroxyacétylamino-5-(1,3,4-trihydroxy-2-butyl)aminocarbonyl-2,4,6-triiodo-benzoyl-amino]-2,2-bis-hydroxyméthyl-propane.
- 1,3-bis-[3-(L-2-hydroxy-propionyl)amino-5-[N-méthyl-N-(1,3-dihydroxy-isopropyl)]aminocarbonyl-2,4,6-triiodo-benzoyl-amino]-2-hydroxy-propane.
- 1,3-bis-[3-(L-2-hydroxy-propionyl)amino-5-[N-méthyl-N-(2,3-dihydroxy-propyl)]aminocarbonyl-2,4,6-triiodo-benzoyl-amino]-2-hydroxy-propane.
- 1,3-bis-[N-méthyl-N-[3-(L-2-hydroxy-propionyl)amino-5-(1,3-dihydroxy-isopropyl)aminocarbonyl-2,4,6-triiodo-benzoyl]amino]-2-hydroxy-propane.
- 1,3-bis-[N-méthyl-N-[3-(L-2-hydroxy-propionyl)amino-5-(2,3-dihydroxy-propyl)aminocarbonyl-2,4,6-triiodo-benzoyl]amino]-2-hydroxy-propane.
- 1,3-bis-[3-hydroxyacétylamino-5-(1,3-dihydroxy-isopropyl)aminocarbonyl-2,4,6-triiodo-benzoyl-amino]-2-hydroxy-propane.
- 1,3-bis-[3-hydroxyacétylamino-5-(2,3-dihydroxy-propyl)aminocarbonyl-2,4,6-triiodo-benzoyl-amino]-2-hydroxy-propane.
- 1,3-bis-[3-hydroxyacétylamino-5-(1,3,4-trihydroxy-2-butyl)aminocarbonyl-2,4,6-triiodo-benzoyl-amino]-2-hydroxy-propane.
- 1,3-bis-[3-hydroxyacétylamino-5-[N-methyl-N-(1,3-dihydroxy-isopropyl)]aminocarbonyl-2,4,6-triiodo-benzoyl-amino]-2-hydroxy-propane.
- 1,3-bis-[3-hydroxyacétylamino-5-[N-méthyl-N-(2,3-dihydroxy-propyl)]aminocarbonyl-2,4,6-triiodobenzoyl-amino]-2-hydroxy-propane.
- 1,3-bis-[N-méthyl-N-[3-hydroxyacétylamino-5-(1,3-dihydroxy-isopropyl)aminocarbonyl-2,4,6-triiodo-benzoyl]amino]-2-hydroxy-propane.
- 1,3-bis-[N-méthyl-N-[3-hydroxyacétylamino-5-(2,3-dihydroxy-propyl)aminocarbonyl-2,4,6-triiodobenzoyl]amino]-2-hydroxy-propane.
- 1,3-bis-[3-(N-méthyl-N-hydroxyacetyl)amino-5-(1,3-dihydroxy-isopropyl)aminocarbonyl-2,4,6-triiodo-benzoyl-amino]-2-hydroxy-propane.
- 1,3-bis-[3-[N-méthyl-N-(L-2-hydroxy-propionyl)]-amino-5-(1,3,4-trihydroxy-2-butyl)aminocarbonyl-2,4,6-triiodo-benzoyl-amino]-2-hydroxy-propane.
- 1,3-bis-[3-[N-méthyl-N-(L-2-hydroxy-propionyl)]-amino-5-(1,3-dihydroxy-isopropyl)aminocarbonyl-2,4,6-triiodo-benzoyl-amino]-2-hydroxy-propane.
- 1,3-bis-[3-[N-méthyl-N-(L-2-hydroxy-propionyl)]-amino-5-(2,3-dihydroxy-propyl)aminocarbonyl-2,4,6-triiodo-benzoyl-amino]-2-hydroxy-propane.
- 1,3-bis-[3-(N-méthyl-N-hydroxyacetyl)amino-5-(2,3-dihydroxy-propyl)aminocarbonyl-2,4,6-triiodo-benzoyl-amino]-2-hydroxy-propane.
- 1,3-bis-[3-(N-méthyl-N-hydroxyacétyl)amino-5-(1,3,4-trihydroxy-2-butyl)aminocarbonyl-2,4,6-triiodo-benzoyl-amino]-2-hydroxy-propane.
- 1,3-bis-[3-(L-2-hydroxy-propionyl)amino-5-(1,3-dihydroxy-isopropyl)aminocarbonyl-2,4,6-triiodo-benzoyl-amino]-2,2-bis-hydroxyméthyl-propane.
- 1,3-bis-[3-(L-2-hydroxy-propionyl)amino-5-(2,3-dihydroxy-propyl)aminocarbonyl-2,4,6-triiodo-benzoyl-amino]-2,2-bis-hydroxyméthyl-propane.
- 1,3-bis-(3-(L-2-hydroxy-propionyl)amino-5-(1,3,4-trihydroxy-2-butyl)aminocarbonyl-2,4,6-triiodo-benzoyl-amino]-2,2-bis-hydroxyméthyl-propane.
- 1,3-bis-[3-[N-méthyl-N-(L-2-hydroxy-propionyl)]-amino-5-(1,3-dihydroxy-isopropyl)aminocarbonyl-2,4,6-triiodo-benzoyl-amino]-2 2-bis-hydroxyméthyl-propane.
- 1,3-bis-[3-[N-méthyl-N-(L-2-hydroxy-propionyl)]-amino-5-(2,3-dihydroxy-propyl)aminocarbonyl-2,4,6-triiodo-benzoyl-amino]-2,2-bis-hydroxyméthyl-propane.
- 1,3-bis-[3-(N-méthyl-N-(L-2-hydroxy-propionyl)]-amino-5-(1,3,4-trihydroxy-2-butyl)aminocarbonyl-2,4 6-triiodo-benzoyl-amino]-2,2-bis-hydroxyméthyl-propane.
- 1,3-bis-[3-(N-méthyl-N-hydroxyacetyl)amino-5-(1,3-dihydroxy-isopropyl)aminocarbonyl-2,4,6-triiodo-benzoyl-amino]-2,2-bis-hydroxyméthyl-propane.
- 1,3-bis-[3-(N-méthyl-N-hydroxyacetyl)amino-5-(2,3-dihydroxy-propyl)aminocarbonyl-2,4,6-triiodo-benzoyl-amino]-2,2-bis-hydroxyméthyl-propane.
- 1,3-bis-[3-(N-méthyl-N-hydroxyacetyl)amino-5-(1,3,4-trihydroxy-2-butyl)aminocarbonyl-2,4,6-triiodo-benzoyl-amino]-2,2-bis-hydroxyméthyl-propane.
- 1,3-bis-[3-(L-2-hydroxy-propionyl)amino-5-[N-méthyl-N-(D-1-déoxy-glucitol)]aminocarbonyl-2,4,6-triiodo-benzoyl-amino]-2-hydroxy-propane.
- 1,3-bis-[3-(L-2-hydroxy-propionyl)amino-5-[N-méthyl-N-(D-1-déoxy-glucitol)]aminocarbonyl-2,4,6-triiodo-benzoyl-amino]-2,2-bis-hydroxyméthyl-propane.
- 1-[3-hydroxyacétylamino-5-(1,3,4-trihydroxy-2-butyl)aminocarbonyl-2,4,6-triiodo-benzoyl-amino]-3-[3-(L-2-hydroxy-propionyl)amino-5-(1,3-dihydroxy-isopropyl)aminocarbonyl-2,4,6-triiodo-benzoyl-amino]-2-hydroxy-propane.
- 1-[3-(L-2-hydroxy-propionyl)amino-5-(1,3-dihydroxy-isopropyl)aminocarbonyl-2,4,6-triiodo-benzoyl-amino]-3-[3-(L-2-hydroxy-propionyl)amino-5-(2,3-dihydroxy-propyl)aminocarbonyl-2,4,6-triiodoben zoyl-amino]-2-hydroxy-propane.
- 1-[3-(L-2-hydroxy-propionyl)amino-5-(1,3-dihydroxy-isopropyl)aminocarbonyl-2,4,6-triiodo-benzoyl-amino]-3-[3-(L-2-hydroxy-propionyl)amino-5-[N-methyl-N-(D-1-déoxy-glucitol)]aminocarbonyl-2,4,6-triiodo-benzoyl-amino]-2-hydroxy-propane.
- 1,3-bis-[N-méthyl-N-[3-hydroxyacétylamino-5-(1,3,4-trihydroxy-2-butyl)aminocarbonyl-2,4,6-triiodo-benzoyl]amino]-2-hydroxy-propane.
- 1,3-bis-[N-méthyl-N-[3-hydroxyacétylamino-5-(N-méthyl-N-(1,3-dihydroxy-isopropyl)]aminocarbonyl-2,4,6-triiodo-benzoyl]amino]-2-hydroxy-propane.
- 1,3-bis-[N-méthyl-N-[3-hydroxyacétylamino-5-[N-méthyl-N-(D-1-déoxy-glucitol)]aminocarbonyl-2,4,6-triiodo-benzoyl]amino]-2-hydroxy-propane.
- 1,3-bis-[N-méthyl-N-[3-(L-2-hydroxy-propionyl)amino-5-(2-hydroxy-éthyl)aminocarbonyl-2,4,6-triiodo-benzoyl]amino]-2-hydroxy-propane.
- 1,3-bis-[N-méthyl-N-[3-(L-2-hydroxy-propionyl)amino-5-(1,3,4-trihydroxy-2-butyl)aminocarbonyl-2,4,6-triiodo-benzoyl]amino]-2-hydroxy-propane.
- 1,3-bis-(N-méthyl-N-[3-[N-méthyl-N-(L-2-hydroxy-propionyl)]amino-5-(2-hydroxy-éthyl)aminocarbonyl-2,4,6-triiodo-benzoyl]amino]-2-hydroxy-propane.
- 1,3-bis-[N-méthyl-N-[3-(N-méthyl-N-(L-2-hydroxy-propionyl)]amino-5-(1,3-dihydroxy-ispropyl)aminocarbonyl-2,4,6-triiodo-benzoyl]amino]-2-hydroxy-propane.

6. Procédé pour la préparation du 1,3-bis[3-(mono-ou poly-hydroxy)acylamino-5-(mono- ou poly-hydroxyalkyl)aminocarbonyl-2,4,6-triiodobenzoylamino]-hydroxy- ou hydroxyalkyl-propane de formule générale (I) selon la revendication 1, caractérisé en ce qu'un 1,3-diamino-hydroxy- ou hydroxyalkyl-propane de formule générale (II)
R₃-HN-CH₂-X-CH₂-NH-R₃' (II)
où R₃ et R₃' sont H ou CH₃, X est l'un des groupes -CH(OH)-, -CH(CH₂OH)-, -C(OH)(CH₂OH)- ou -C(CH₂OH)₂- et l'un des groupes amino peut être protégé par un groupe protecteur approprié, est amené à réagir, directement ou par un procédé à plusieurs étapes, avec un dérivé réactif d'un acide 3-(mono- ou poly-acyloxy)acylamino-5-(mono ou poly-hydroxyalkyl)aminocarbonyl-2,4,6-triiodobenzoïque de formule générale (III) 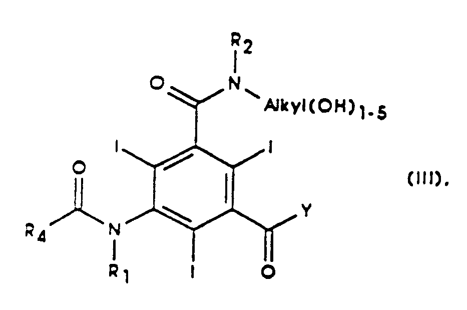 où :
R₁, R₂ représentent H ou CH₃,
R₄ est un résidu mono- ou poly-acyloxy(alkyle en C₁-C₁₃) à chaîne droite ou ramifiée, contenant 3 à 13 atomes de C et 1 ou 2 groupes acyloxy inférieurs en C₂-C₅,
Alkyl(OH)₁₋₅ est l'un des groupes de formule -CH₂(CHOH)₄CH₂OH, -CH₂CH(OH)CH₂OH, -CH(CH₂OH)CH(OH)CH₂OH, -CH₂CH₂OH, -CH(CH₂OH)₂, ou dans lequel les groupes hydroxyle peuvent être protégés, de préférence par des groupes de type acétal ou cétal,
CO-Y est le résidu d'un anhydride mixte ou, de préférence, un groupe halogénocarbonyle,
pour donner le produit de formule générale (IV) 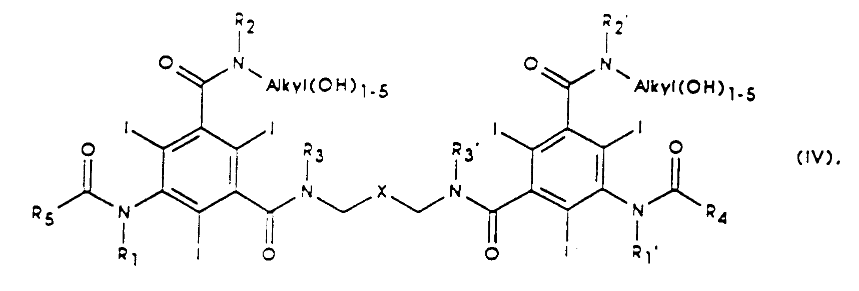 où R₁, R₂, R₃, R₁', R₂', R₃', R₄, Alkyl(OH)₁₋₅ et X sont tels que définis précédemment et R₅ peut être R ou R₄, selon l'un des modes opératoires suivants :
a) un composé de formule générale (II) est amené à réagir avec un composé de formule générale (III) en un rapport molaire de 1:2 dans un solvant et en présence d'un agent de condensation basique pour produire directement un composé de formule (IV) où R₅ correspond à R₄,
b) un composé de formule (II), où l'un des deux groupes amino est protégé par un groupe protecteur approprié, est amené à réagir avec un composé de formule (III) en un rapport molaire de 1:1 dans un solvant et en présence d'un agent de condensation basique pour donner, après hydrolyse des groupes protecteurs, le dérivé 1-[3-(mono- ou poly-hydroxy)acylamino-5-(mono- ou poly-hydroxyalkyl)aminocarbonyl-2,4,6-triiodobenzoylamino]-3-amino-hydroxyalkylé de formule (V) 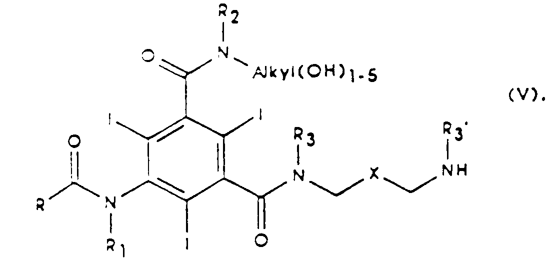 où R, R₁, R₂, R₃, R₃', Alkyl(OH)₁₋₅ et X sont tels que définis précédemment, et ce composé (V) est ensuite amené à réagir avec un dérivé de formule (III) en présence d'un agent de condensation basique pour produire le composé (IV) désiré où R₅ correspond à R,
puis le composé (IV) obtenu par l'un des modes de synthèse a) ou b) est transformé, par hydrolyse des groupes protecteurs, en le composé correspondant de formule (I), et ce dernier, si R₁ et R₁' sont tous deux H, peut être facultativement N-méthylé dans un milieu alcalin.

7. Procédé pour la préparation de composés de formule générale (I) où R₁ et R₁' sont CH₃, caractérisé en ce qu'un composé de formule générale (I) dans lequel R₁ et R₁' sont l'hydrogène est méthylé dans une solution alcaline par traitement avec un halogénure de méthyle, le sulfate de diméthyle, un méthylsulfonate, le carbonate de diméthyle.

8. Agents de contraste radiologique non ioniques contenant, comme constituant opacifiant, au moins un 1,3-bis[3-(mono- ou poly-hydroxy)acylamino-5-(mono- ou poly-hydroxyalkyl)aminocarbonyl-2,4,6-triiodobenzoylamino]-hydroxy ou hydroxyalkyl-propane selon les revendications 1 à 5.

## Revendications (Revendications pour l'(les) Etat(s) contractant(s) suivant(s): ES, GR)

1. Procédé de préparation de 1,3-bis[3-(mono- ou poly-hydroxy)acylamino-5-(mono- ou poly-hydroxyalkyl)aminocarbonyl-2,4,6-triiodobenzoylamino]-hydroxy- ou hydroxyalkyl-propanes symétriques et asymétriques de formule générale (I) 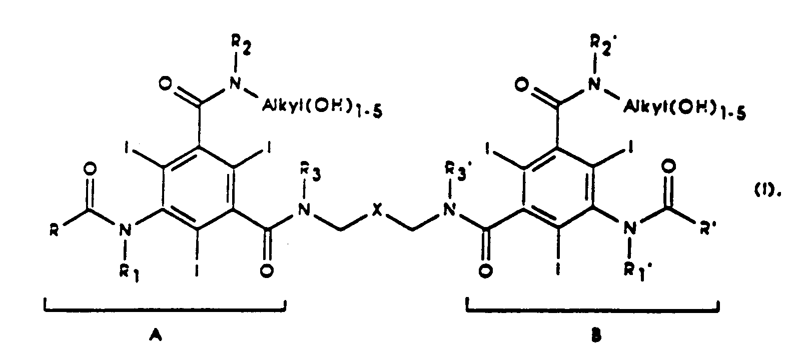 où :
R, R', qui sont identiques ou différents, représentent un résidu mono- ou polyhydroxyalkyle en C₁-C₃ à chaîne droite ou ramifiée contenant 1 ou 2 groupes OH,
R₁, R₂, R₃, qui sont identiques ou différents, sont H ou CH₃,
R₁', R₂', R₃', qui sont identiques ou différents, sont H ou CH₃,
Alkyl(OH)₁₋₅ est l'un des groupes de formule -CH(CH₂OH)CH(OH)CH₂OH, -CH(CH₂OH)₂, -CH₂CH(OH)CH₂OH, -CH₂(CHOH)₄CH₂OH ou -CH₂CH₂OH,
X est l'un des groupes -CH(OH)-, -CH(CH₂OH)-, -C(OH)(CH₂OH)- ou -C(CH₂OH)₂-,
A et B, peuvent être identiques ou différents, leurs énantiomères, diastéréoisomères et/ou rotamères possibles, caractérisé en ce qu'un 1,3-diamino-hydroxy- ou hydroxyalkyl -propane de formule générale (II)
R₃-HN-CH₂-X-CH₂-NH-R₃' (II)
où R₃ et R₃' sont H ou CH₃, X est l'un des groupes -CH(OH)-, -CH(CH₂OH)-, -C(OH)(CH₂OH)- ou -C(CH₂OH)₂- et l'un des groupes amino peut être protégé par un groupe protecteur approprié, est amené à réagir, directement ou par un procédé à plusieurs étapes, avec un dérivé réactif d'un acide 3-(mono- ou poly-acyloxy)acylamino-5-(mono- ou poly-hydroxyalkyl)aminocarbonyl-2,4,6-triiodobenzoïque de formule générale (III) 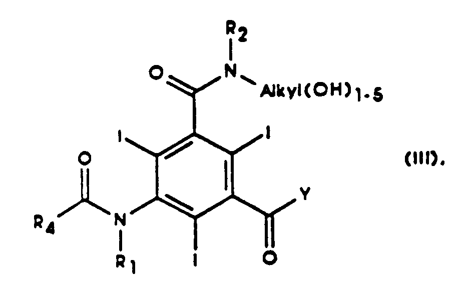 où :
R₁, R₂ représentent H ou CH₃,
R₄ est un résidu mono- ou poly-acyloxy(alkyle en C₁-C₁₃) à chaîne droite ou ramifiée, contenant 3 à 13 atomes de C et 1 ou 2 groupes acyloxy inférieurs en C₂-C₅,
Alkyl(OH)₁₋₅ est l'un des groupes de formule -CH₂(CHOH)₄CH₂OH, -CH₂CH(OH)CH₂OH, -CH(CH₂OH)CH(OH)CH₂OH, -CH₂CH₂OH, -CH(CH₂OH)₂, ou dans lequel les groupes hydroxyle peuvent être protégés, de préférence par des groupes de type acétal ou cétal,
CO-Y est le résidu d'un anhydride mixte ou, de préférence, un groupe halogénocarbonyle,
pour donner le produit de formule générale (IV) 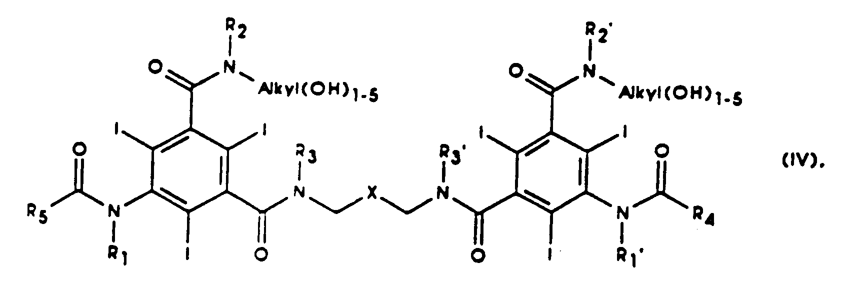 où R₁, R₂, R₃, R₁', R₂', R₃', R₄, Alkyl(OH)₁₋₅ et X sont tels que définis précédemment et R₅ peut être R ou R₄, selon l'un des modes opératoires suivants :
a) un composé de formule générale (II) est amené à réagir avec un composé de formule générale (III) en un rapport molaire de 1:2 dans un solvant et en présence d'un agent de condensation basique pour produire directement un composé de formule (IV) où R₅ correspond à R₄,
b) un composé de formule (II), où l'un des deux groupes amino est protégé par un groupe protecteur approprié, est amené à réagir avec un composé de formule (III) en un rapport molaire de 1:1 dans un solvant et en présence d'un agent de condensation basique pour donner, après hydrolyse des groupes protecteurs, le dérivé 1-[3-(mono- ou poly-hydroxy)acylamino-5-(mono- ou poly-hydroxyalkyl)aminocarbonyl-2,4,6-triiodobenzoylamino]-3-amino-hydroxyalkylé de formule (V) 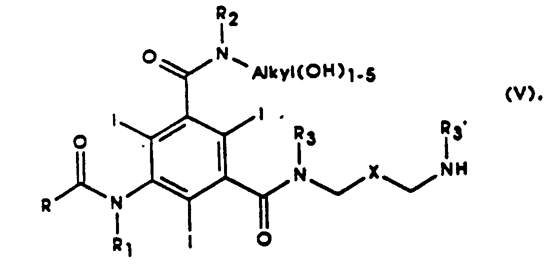 où R, R₁, R₂, R₃, R₃', Alkyl(OH)₁₋₅ et X sont tels que définis précédemment, et ce composé (V) est ensuite amené à réagir avec un dérivé de formule (III) en présence d'un agent de condensation basique pour produire le composé (IV) désiré où R₅ correspond à R,
puis le composé (IV) obtenu par l'un des modes de synthèse a) ou b) est transformé, par hydrolyse des groupes protecteurs, en le composé correspondant de formule (I), et ce dernier, si R₁ et R₁' sont tous deux H, peut être facultativement N-méthylé dans un milieu alcalin.

2. Procédé pour la préparation de composés de formule générale (I) où R₁ et R₁' sont CH₃, caractérisé en ce qu'un composé de formule générale (I) dans lequel R₁ et R₁' sont l'hydrogène est méthylé dans une solution alcaline par traitement avec un halogénure de méthyle, le sulfate de diméthyle, un méthylsulfonate, le carbonate de diméthyle.
